# EUROPEAN PATENT APPLICATION

(11) **EP 1 489 177 A1**
(43) Date of publication of application: **22.12.2004**
(21) Application number: 03710315.7
(22) Date of filing: 12.03.2003
(51) Int. Cl.: C12N 15/12, C07K 14/47, C07K 16/18, C12Q 1/48, C12P 21/02, G01N 33/50, G01N 33/15, G01N 33/53, A61K 45/00, A61K 38/00, A61K 39/395, A61P 35/00, A61P 43/00

(54) **CDC7-ASK KINASE COMPLEX, SUBSTRATE OF THE KINASE COMPLEX, ANTIBODY SPECIFIC TO THE SUBSTRATE, AND METHOD OF SCREENING COMPOUND CAPABLE OF INHIBITING CDC7-ASK KINASE USING THE SAME**

(30) Priority: 12.03.2002 JP 2002067702
(71) Applicant: Japan Science and Technology Agency, Kawaguchi-shi, Saitama 332-0012 (JP); Ginkgo Biomedical Research Institute Co., Ltd., Tokyo 108-0071 (JP); Medical and Biological Laboratories Co., Ltd., Nagoya-shi, Aichi 460-0002 (JP)
(72) Inventor: MASAI, Hisao, Tokyo 108-0073 (JP); TAMAI, Katsuyuki, Ina-shi, Nagano 396-0111 (JP)
(74) Representative: VOSSIUS & PARTNER
(86) International application number: PCT/JP2003/002918
(87) International publication number: WO 2003/076623

(57) **Abstract**

The present invention provides methods for measuring the phosphorylation activity of Cdc7-ASK kinase complex by using as an indicator the level of phosphorylation at a phosphorylation site of MCM, which is a substrate of Cdc7-ASK kinase complex. The effects of test compounds on the phosphorylation activity of Cdc7-ASK kinase complex can also be evaluated based on these measurement methods. Compounds that inhibit this phosphorylation activity are useful as anti-cancer agents having superior specificity for cancer.

SEQUENCE LISTING

<110> JAPAN SCIENCE AND TECHNOLOGY AGENCY
GINKGO BIOMEDICAL RESEARCH INSTITUTE CO., LTD
MEDICAL AND BIOLOGICAL LABORATORIES. CO., LTD.

<120> A Cdc7-ASK kinase complex, a substrate thereof, a
specific antibody thereof, and a method for screening
of a compound having the activity which inhibit the
Cdc7-ASK kinase using them.

<130> M3-A0201P

<140>
<141>

<150> JP 2002-067702
<151> 2002-03-12

<160> 21

<170> PatentIn Ver. 2.1

<210> 1
<211> 130
<212> PRT

<213> Homo sapiens

<400> 1
   Met Ala Ser Ser Pro Ala Gln Arg Arg Arg Gly Asn Asp Pro Leu Thr
   1               5                   10                  15
   Ser Ser Pro Gly Arg Ser Ser Arg Arg Thr Asp Ala Leu Thr Ser Ser
               20                  25                  30
   Pro Gly Arg Asp Leu Pro Pro Phe Glu Asp Glu Ser Glu Gly Leu Leu
           35                  40                  45
   Gly Thr Glu Gly Pro Leu Glu Glu Glu Glu Asp Gly Glu Glu Leu Ile
       50                  55                  60
   Gly Asp Gly Met Glu Arg Asp Tyr Arg Ala Ile Pro Glu Leu Asp Ala
   65                  70                  75                  80
   Tyr Glu Ala Glu Gly Leu Ala Leu Asp Asp Glu Asp Val Glu Glu Leu
                   85                  90                  95
   Thr Ala Ser Gln Arg Glu Ala Ala Glu Arg Ala Met Arg Gln Arg Asp
               100                 105                 110
   Arg Glu Ala Gly Arg Gly Leu Gly Arg Met Arg Arg Gly Leu Leu Tyr
           115                 120                 125
   Asp Ser
   130

<210> 2

<211> 3379

<212> DNA

<213> Homo sapiens

<220>

<221> CDS

<222> (31)..(2709)

<400> 2

aattccgcgg aatcatcgga atccttcacc atg gca tcc agc ccg gcc cag cgt 54

Met Ala Ser Ser Pro Ala Gln Arg

1               5

cgg cga ggc aat gat cct ctc acc tcc agc cct ggc cga agc tcc cgg 102

Arg Arg Gly Asn Asp Pro Leu Thr Ser Ser Pro Gly Arg Ser Ser Arg

    10                  15                  20

cgt act gat gcc ctc acc tcc agc cct ggc cgt gac ctt cca cca ttt 150

Arg Thr Asp Ala Leu Thr Ser Ser Pro Gly Arg Asp Leu Pro Pro Phe

25                  30                  35                  40

gag gat gag tcc gag ggg ctc cta ggc aca gag ggg ccc ctg gag gaa 198
Glu Asp Glu Ser Glu Gly Leu Leu Gly Thr Glu Gly Pro Leu Glu Glu
                45                  50                  55

gaa gag gat gga gag gag ctc att gga gat ggc atg gaa agg gac tac 246
Glu Glu Asp Gly Glu Glu Leu Ile Gly Asp Gly Met Glu Arg Asp Tyr
            60                  65                  70

cgc gcc atc cca gag ctg gac gcc tat gag gcc gag gga ctg gct ctg 294
Arg Ala Ile Pro Glu Leu Asp Ala Tyr Glu Ala Glu Gly Leu Ala Leu
        75                  80                  85

gat gat gag gac gta gag gag ctg acg gcc agt cga agg gag gca gca 342
Asp Asp Glu Asp Val Glu Glu Leu Thr Ala Ser Arg Arg Glu Ala Ala
    90                  95                  100

gac ggg cca tgc ggc acg gtg acc ggg agc tgg ccg ggg ctg ggc gca 390
Asp Gly Pro Cys Gly Thr Val Thr Gly Ser Trp Pro Gly Leu Gly Ala
105                 110                 115                 120

tgc gcc gtg ggc tcc tgt atg aca gcg atg agg agg acg agg agc gcc 438
Cys Ala Val Gly Ser Cys Met Thr Ala Met Arg Arg Thr Arg Ser Ala
                125                 130                 135

ctg ccc gca agc gcc gcc agt gga gcc ggc acg gag gac ggc gag gag 486

Leu Pro Ala Ser Ala Ala Ser Gly Ala Gly Thr Glu Asp Gly Glu Glu
            140                 145                 150

gac gag cag atg att gag agc atc gag aac ctg gag gat ctc aaa ggc 534
Asp Glu Gln Met Ile Glu Ser Ile Glu Asn Leu Glu Asp Leu Lys Gly
        155                 160                 165

cac tct gtg cgc gag tgg gtg agc atg gcg ggc ccc cgg ctg gag atc 582
His Ser Val Arg Glu Trp Val Ser Met Ala Gly Pro Arg Leu Glu Ile
    170                 175                 180

cac cac cgc ttc aag aac ttc ctg cgc act cac gtc gac agc cac ggc 630
His His Arg Phe Lys Asn Phe Leu Arg Thr His Val Asp Ser His Gly
185                 190                 195                 200

cac aac gtc ttc aag gag cgc atc agc gac atg tgc aaa gag aac cgt 678
His Asn Val Phe Lys Glu Arg Ile Ser Asp Met Cys Lys Glu Asn Arg
                205                 210                 215

gag agc ctg gtg gtg aac tat gag gac ttg gca gcc agg gag cac gtg 726
Glu Ser Leu Val Val Asn Tyr Glu Asp Leu Ala Ala Arg Glu His Val
            220                 225                 230

ctg gcc tac ttc ctg cct gag gca ccg gcg gag ctg ctg cag atc ttt 774
Leu Ala Tyr Phe Leu Pro Glu Ala Pro Ala Glu Leu Leu Gln Ile Phe
        235                 240                 245

gat gag gct gcc ctg gag gtg gta ctg gcc atg tac ccc aag tac gac 822
Asp Glu Ala Ala Leu Glu Val Val Leu Ala Met Tyr Pro Lys Tyr Asp
    250                 255                 260

cgc atc acc aac cac atc cat gtc cgc atc tcc cac ctg cct ctg gtg 870
Arg Ile Thr Asn His Ile His Val Arg Ile Ser His Leu Pro Leu Val
265                 270                 275                 280

gag gag ctg cgc tcg ctg agg cag ctg cat ctg aac cag ctg atc cgc 918
Glu Glu Leu Arg Ser Leu Arg Gln Leu His Leu Asn Gln Leu Ile Arg
                285                 290                 295

acc agt ggg gtg gtg acc agc tgc act ggc gtc ctg ccc cag ctc agc 966
Thr Ser Gly Val Val Thr Ser Cys Thr Gly Val Leu Pro Gln Leu Ser
            300                 305                 310

atg gtc aag tac aac tgc aac aag tgc aat ttc gtc ctg ggt cct ttc 1014
Met Val Lys Tyr Asn Cys Asn Lys Cys Asn Phe Val Leu Gly Pro Phe
        315                 320                 325

tgc cag tcc cag aac cag gag gtg aaa cca ggc tcc tgt cct gag tgc 1062
Cys Gln Ser Gln Asn Gln Glu Val Lys Pro Gly Ser Cys Pro Glu Cys
    330                 335                 340

cag tcg gcc ggc ccc ttt gag gtc aac atg gag gag acc atc tat cag 1110

Gln Ser Ala Gly Pro Phe Glu Val Asn Met Glu Glu Thr Ile Tyr Gln
345                 350                 355                 360

aac tac cag cgt atc cga atc cag gag agt cca ggc aaa gtg gcg gct 1158
Asn Tyr Gln Arg Ile Arg Ile Gln Glu Ser Pro Gly Lys Val Ala Ala
                365                 370                 375

cgg cgg ctg ccc cgc tcc aag gac gcc att ctc ctc gca gat ctg gtg 1206
Arg Arg Leu Pro Arg Ser Lys Asp Ala Ile Leu Leu Ala Asp Leu Val
            380                 385                 390

gac agc tgc aac gca gga gac gag ata gag ctg act ggc atc tat cac 1254
Asp Ser Cys Asn Ala Gly Asp Glu Ile Glu Leu Thr Gly Ile Tyr His
        395                 400                 405

aac aac tat gat ggc tcc ctc aac act gcc aat ggc ttc cct gtc ttt 1302
Asn Asn Tyr Asp Gly Ser Leu Asn Thr Ala Asn Gly Phe Pro Val Phe
    410                 415                 420

gcc act gtc atc cta gcc aac cac gtg gcc aag aag gac aac aag gtt 1350
Ala Thr Val Ile Leu Ala Asn His Val Ala Lys Lys Asp Asn Lys Val
425                 430                 435                 440

gct gta ggg gaa ctg acc gat gaa gat gtg aag atg atc act agc ctc 1398
Ala Val Gly Glu Leu Thr Asp Glu Asp Val Lys Met Ile Thr Ser Leu
                445                 450                 455

tcc aag gat cag cag atc gga gag aag atc ttt gcc agc att gct cct 1446
Ser Lys Asp Gln Gln Ile Gly Glu Lys Ile Phe Ala Ser Ile Ala Pro
            460                 465                 470

tcc atc tat ggt cat gaa gac atc aag aga ggc cct gct ctg gcc ctg 1494
Ser Ile Tyr Gly His Glu Asp Ile Lys Arg Gly Pro Ala Leu Ala Leu
        475                 480                 485

ttc gga ggg gag ccc aaa aac cca ggt ggc aag cac aag gta cgt ggt 1542
Phe Gly Gly Glu Pro Lys Asn Pro Gly Gly Lys His Lys Val Arg Gly
    490                 495                 500

gat atc aac gtg ctc ttg tgc gga gac cct ggc aca gcg aag tcg cag 1590
Asp Ile Asn Val Leu Leu Cys Gly Asp Pro Gly Thr Ala Lys Ser Gln
505                 510                 515                 520

ttt ctc aag tat att gag aaa gtg tcc agc cga gcc atc ttc acc act 1638
Phe Leu Lys Tyr Ile Glu Lys Val Ser Ser Arg Ala Ile Phe Thr Thr
                525                 530                 535

ggc cag ggg gcg tcg gct gtg gcc gtc acg gcg tat gtc cag cgg cac 1686
Gly Gln Gly Ala Ser Ala Val Ala Val Thr Ala Tyr Val Gln Arg His
            540                 545                 550

cct gtc agc agg gag tgg acc ttg gag gct ggg gcc ctg gtt ctg gct 1734

Pro Val Ser Arg Glu Trp Thr Leu Glu Ala Gly Ala Leu Val Leu Ala
        555                 560                 565

gac cga gga gtg tgt ctc att gat gaa ttt gac aag atg aat gac cag 1782
Asp Arg Gly Val Cys Leu Ile Asp Glu Phe Asp Lys Met Asn Asp Gln
    570                 575                 580

gac aga acc agc atc cat gag gcc atg gag caa cag agc atc tcc atc 1830
Asp Arg Thr Ser Ile His Glu Ala Met Glu Gln Gln Ser Ile Ser Ile
585                 590                 595                 600

tcg aag gct ggc atc gtc acc tcc ctg cag gct cgc tgc acg gtc att 1878
Ser Lys Ala Gly Ile Val Thr Ser Leu Gln Ala Arg Cys Thr Val Ile
                605                 610                 615

gct gcc gcc aac ccc ata gga ggg cgc tac gac ccc tcg ctg act ttc 1926
Ala Ala Ala Asn Pro Ile Gly Gly Arg Tyr Asp Pro Ser Leu Thr Phe
            620                 625                 630

tct gag aac gtg gac ctc aca gag ccc atc atc tca cgc ttt gac atc 1974
Ser Glu Asn Val Asp Leu Thr Glu Pro Ile Ile Ser Arg Phe Asp Ile
        635                 640                 645

ctg tgt gtg gtg agg gac acc gtg gac cca gtc cag gac gag atg ctg 2022
Leu Cys Val Val Arg Asp Thr Val Asp Pro Val Gln Asp Glu Met Leu
    650                 655                 660

gcc cgc ttc gtg gtg ggc agc cac gtc aga cac cac ccc agc aac aag 2070
Ala Arg Phe Val Val Gly Ser His Val Arg His His Pro Ser Asn Lys
665                 670                 675                 680

gag gag gag ggg ctg gcc aat ggc agc gct gct gag ccc gcc atg ccc 2118
Glu Glu Glu Gly Leu Ala Asn Gly Ser Ala Ala Glu Pro Ala Met Pro
                685                 690                 695

aac acg tat ggc gtg gag ccc ctg ccc cag gag gtc ctg aag aag tac 2166
Asn Thr Tyr Gly Val Glu Pro Leu Pro Gln Glu Val Leu Lys Lys Tyr
            700                 705                 710

atc atc tac gcc aag gag agg gtc cac ccg aag ctc aac cag atg gac 2214
Ile Ile Tyr Ala Lys Glu Arg Val His Pro Lys Leu Asn Gln Met Asp
        715                 720                 725

cag gac aag gtg gcc aag atg tac agt gac ctg agg aaa gaa tct atg 2262
Gln Asp Lys Val Ala Lys Met Tyr Ser Asp Leu Arg Lys Glu Ser Met
    730                 735                 740

gcg aca ggc agc atc ccc att acg gtg cgg cac atc gag tcc atg agt 2310
Ala Thr Gly Ser Ile Pro Ile Thr Val Arg His Ile Glu Ser Met Ser
745                 750                 755                 760

cat ggc gga ggc cca cgc gcg cat cca tct gcg gga cta tgt gat cga 2358

His Gly Gly Gly Pro Arg Ala His Pro Ser Ala Gly Leu Cys Asp Arg
                765                 770                 775

aga cga cgt caa cat ggc cat ccg cgt gat gct gga gag ctt cat aga 2406
Arg Arg Arg Gln His Gly His Pro Arg Asp Ala Gly Glu Leu His Arg
            780                 785                 790

cac aca gaa gtt cag cgt cat cgc agc atg cgc aag act ttt gcc cgc 2454
His Thr Glu Val Gln Arg His Arg Ser Met Arg Lys Thr Phe Ala Arg
        795                 800                 805

tac ctt tca ttc cgg cgt gac aac aat gag ctg ttg ctc ttc ata ctg 2502
Tyr Leu Ser Phe Arg Arg Asp Asn Asn Glu Leu Leu Leu Phe Ile Leu
    810                 815                 820

aag cag tta gtg gca gag cag gtg aca tat cag cgc aac cgc ttt ggg 2550
Lys Gln Leu Val Ala Glu Gln Val Thr Tyr Gln Arg Asn Arg Phe Gly
825                 830                 835                 840

gcc cag cag gac act att gag gtc cct gag aag gac ttg gtg gat aag 2598
Ala Gln Gln Asp Thr Ile Glu Val Pro Glu Lys Asp Leu Val Asp Lys
                845                 850                 855

gct cgt cag atc aac atc cac aac ctc tct gca ttt tat gac agt gag 2646
Ala Arg Gln Ile Asn Ile His Asn Leu Ser Ala Phe Tyr Asp Ser Glu
            860                 865                 870

ctc ttc agg atg aac aag ttc agc cac gac ctg aaa agg aaa atg atc 2694
Leu Phe Arg Met Asn Lys Phe Ser His Asp Leu Lys Arg Lys Met Ile
        875                 880                 885

ctg cag cag ttc tga ggccctatgc catccataag gattccttgg gattctggtt 2749
Leu Gln Gln Phe
    890

tggggtggtc agtgccctct gtgctttatg gacacaaaac cagagcactt gatgaactcg 2809
gggtactagg gtcagggctt atagcaggat gtctggctgc acctggcatg actgtttgtt 2869
tctccaagcc tgctttgtgc ttctcacctt tgggtgggat gccttgccag tgtgtcttac 2929
ttggttgctg aacatcttgc cacctccgag tgctttgtct ccactcagta ccttggatca 2989
gagctgctga gttcaggatg cctgcgtgtg gtttaggtgt tagccttctt acatggatgt 3049
caggagagct gctgccctct tggcgtgagt tgcgtattca ggctgctttt gctcgctttg 3109
gccagagagc tggttgaaga tgtttgtaat cgttttcagt ctcctgcagg tttctgtgcc 3169
cctgtggtgg aagaggcacg acagtgccag cgcagcgttc tgggctcctc agtcgcaggg 3229
gtgggatgtg agtcatgcgg attatccact cgccacagtt atcagctgcc attgctccct 3289
gtctgtttcc ccactctctt atttgtgcat tcggtttggt ttctgtagtt ttaattttta 3349
ataaagttga ataaaatata aaaaaaaaaa 3379

<210> 3
<211> 892
<212> PRT
<213> Homo sapiens

<400> 3

Met Ala Ser Ser Pro Ala Gln Arg Arg Arg Gly Asn Asp Pro Leu Thr

1               5                   10                  15

Ser Ser Pro Gly Arg Ser Ser Arg Arg Thr Asp Ala Leu Thr Ser Ser

            20                  25                  30

Pro Gly Arg Asp Leu Pro Pro Phe Glu Asp Glu Ser Glu Gly Leu Leu

        35                  40                  45

Gly Thr Glu Gly Pro Leu Glu Glu Glu Glu Asp Gly Glu Glu Leu Ile

    50                  55                  60

Gly Asp Gly Met Glu Arg Asp Tyr Arg Ala Ile Pro Glu Leu Asp Ala

65                  70                  75                  80

Tyr Glu Ala Glu Gly Leu Ala Leu Asp Asp Glu Asp Val Glu Glu Leu

                85                  90                  95

Thr Ala Ser Arg Arg Glu Ala Ala Asp Gly Pro Cys Gly Thr Val Thr

            100                 105                 110

Gly Ser Trp Pro Gly Leu Gly Ala Cys Ala Val Gly Ser Cys Met Thr

        115                 120                 125

Ala Met Arg Arg Thr Arg Ser Ala Leu Pro Ala Ser Ala Ala Ser Gly

    130                 135                 140

Ala Gly Thr Glu Asp Gly Glu Glu Asp Glu Gln Met Ile Glu Ser Ile

145                 150                 155                 160

Glu Asn Leu Glu Asp Leu Lys Gly His Ser Val Arg Glu Trp Val Ser

                165                 170                 175

Met Ala Gly Pro Arg Leu Glu Ile His His Arg Phe Lys Asn Phe Leu

            180                 185                 190

Arg Thr His Val Asp Ser His Gly His Asn Val Phe Lys Glu Arg Ile
        195                 200                 205
Ser Asp Met Cys Lys Glu Asn Arg Glu Ser Leu Val Val Asn Tyr Glu
    210                 215                 220
Asp Leu Ala Ala Arg Glu His Val Leu Ala Tyr Phe Leu Pro Glu Ala
225                 230                 235                 240
Pro Ala Glu Leu Leu Gln Ile Phe Asp Glu Ala Ala Leu Glu Val Val
                245                 250                 255
Leu Ala Met Tyr Pro Lys Tyr Asp Arg Ile Thr Asn His Ile His Val
            260                 265                 270
Arg Ile Ser His Leu Pro Leu Val Glu Glu Leu Arg Ser Leu Arg Gln
        275                 280                 285
Leu His Leu Asn Gln Leu Ile Arg Thr Ser Gly Val Val Thr Ser Cys
    290                 295                 300
Thr Gly Val Leu Pro Gln Leu Ser Met Val Lys Tyr Asn Cys Asn Lys
305                 310                 315                 320
Cys Asn Phe Val Leu Gly Pro Phe Cys Gln Ser Gln Asn Gln Glu Val
                325                 330                 335
Lys Pro Gly Ser Cys Pro Glu Cys Gln Ser Ala Gly Pro Phe Glu Val
            340                 345                 350
Asn Met Glu Glu Thr Ile Tyr Gln Asn Tyr Gln Arg Ile Arg Ile Gln
        355                 360                 365
Glu Ser Pro Gly Lys Val Ala Ala Arg Arg Leu Pro Arg Ser Lys Asp
    370                 375                 380
Ala Ile Leu Leu Ala Asp Leu Val Asp Ser Cys Asn Ala Gly Asp Glu
385                 390                 395                 400

Ile Glu Leu Thr Gly Ile Tyr His Asn Asn Tyr Asp Gly Ser Leu Asn
                405                 410                 415
Thr Ala Asn Gly Phe Pro Val Phe Ala Thr Val Ile Leu Ala Asn His
            420                 425                 430
Val Ala Lys Lys Asp Asn Lys Val Ala Val Gly Glu Leu Thr Asp Glu
        435                 440                 445
Asp Val Lys Met Ile Thr Ser Leu Ser Lys Asp Gln Gln Ile Gly Glu
    450                 455                 460
Lys Ile Phe Ala Ser Ile Ala Pro Ser Ile Tyr Gly His Glu Asp Ile
465                 470                 475                 480
Lys Arg Gly Pro Ala Leu Ala Leu Phe Gly Gly Glu Pro Lys Asn Pro
                485                 490                 495
Gly Gly Lys His Lys Val Arg Gly Asp Ile Asn Val Leu Leu Cys Gly
            500                 505                 510
Asp Pro Gly Thr Ala Lys Ser Gln Phe Leu Lys Tyr Ile Glu Lys Val
        515                 520                 525
Ser Ser Arg Ala Ile Phe Thr Thr Gly Gln Gly Ala Ser Ala Val Ala
    530                 535                 540
Val Thr Ala Tyr Val Gln Arg His Pro Val Ser Arg Glu Trp Thr Leu
545                 550                 555                 560
Glu Ala Gly Ala Leu Val Leu Ala Asp Arg Gly Val Cys Leu Ile Asp
                565                 570                 575
Glu Phe Asp Lys Met Asn Asp Gln Asp Arg Thr Ser Ile His Glu Ala
            580                 585                 590
Met Glu Gln Gln Ser Ile Ser Ile Ser Lys Ala Gly Ile Val Thr Ser
        595                 600                 605

Leu Gln Ala Arg Cys Thr Val Ile Ala Ala Ala Asn Pro Ile Gly Gly
    610                 615                 620
Arg Tyr Asp Pro Ser Leu Thr Phe Ser Glu Asn Val Asp Leu Thr Glu
625                 630                 635                 640
Pro Ile Ile Ser Arg Phe Asp Ile Leu Cys Val Val Arg Asp Thr Val
                645                 650                 655
Asp Pro Val Gln Asp Glu Met Leu Ala Arg Phe Val Val Gly Ser His
            660                 665                 670
Val Arg His His Pro Ser Asn Lys Glu Glu Glu Gly Leu Ala Asn Gly
        675                 680                 685
Ser Ala Ala Glu Pro Ala Met Pro Asn Thr Tyr Gly Val Glu Pro Leu
    690                 695                 700
Pro Gln Glu Val Leu Lys Lys Tyr Ile Ile Tyr Ala Lys Glu Arg Val
705                 710                 715                 720
His Pro Lys Leu Asn Gln Met Asp Gln Asp Lys Val Ala Lys Met Tyr
                725                 730                 735
Ser Asp Leu Arg Lys Glu Ser Met Ala Thr Gly Ser Ile Pro Ile Thr
            740                 745                 750
Val Arg His Ile Glu Ser Met Ser His Gly Gly Gly Pro Arg Ala His
        755                 760                 765
Pro Ser Ala Gly Leu Cys Asp Arg Arg Arg Arg Gln His Gly His Pro
    770                 775                 780
Arg Asp Ala Gly Glu Leu His Arg His Thr Glu Val Gln Arg His Arg
785                 790                 795                 800
Ser Met Arg Lys Thr Phe Ala Arg Tyr Leu Ser Phe Arg Arg Asp Asn
                805                 810                 815

Asn Glu Leu Leu Leu Phe Ile Leu Lys Gln Leu Val Ala Glu Gln Val
            820                 825                 830
Thr Tyr Gln Arg Asn Arg Phe Gly Ala Gln Gln Asp Thr Ile Glu Val
        835                 840                 845
Pro Glu Lys Asp Leu Val Asp Lys Ala Arg Gln Ile Asn Ile His Asn
    850                 855                 860
Leu Ser Ala Phe Tyr Asp Ser Glu Leu Phe Arg Met Asn Lys Phe Ser
865                 870                 875                 880
His Asp Leu Lys Arg Lys Met Ile Leu Gln Gln Phe
                885                 890

<210> 4
<211> 2715
<212> DNA
<213> Mus musculus

<220>
<221> CDS
<222> (1)..(2715)

<400> 4
atg gcg gag tct tct gag tct ctc tca gca tct agc cct gcc cgt cag    48
Met Ala Glu Ser Ser Glu Ser Leu Ser Ala Ser Ser Pro Ala Arg Gln
  1               5                  10                  15

cgg cgc cgg atc agt gat ccc ctc acc tcc agc cca ggc cgc agc tcc 96
Arg Arg Arg Ile Ser Asp Pro Leu Thr Ser Ser Pro Gly Arg Ser Ser
            20                  25                  30

aga cgt gct gac gcc ctg acc tcc agc cct ggc aga gac ctc ccc cca 144
Arg Arg Ala Asp Ala Leu Thr Ser Ser Pro Gly Arg Asp Leu Pro Pro
        35                  40                  45

ttt gaa gat gag tct gag ggg ctt ctg ggc aca gag ggg ccc atg gag 192
Phe Glu Asp Glu Ser Glu Gly Leu Leu Gly Thr Glu Gly Pro Met Glu
    50                  55                  60

gaa gaa gag gat gga gag gaa ctc att ggt gat ggc atg gag aga gac 240
Glu Glu Glu Asp Gly Glu Glu Leu Ile Gly Asp Gly Met Glu Arg Asp
65                  70                  75                  80

tac cgt ccc att ccg gag ctc gat gtc tac gag gcc gag gga ttg gcc 288
Tyr Arg Pro Ile Pro Glu Leu Asp Val Tyr Glu Ala Glu Gly Leu Ala
                85                  90                  95

ctg gat gat gaa gat gtg gag gag ctg aca gcc agt cag aga gag gca 336
Leu Asp Asp Glu Asp Val Glu Glu Leu Thr Ala Ser Gln Arg Glu Ala
            100                 105                 110

gct gag cgg acc atg agg cag cgg gac cgt gag gct ggc aga ggc ctg 384
Ala Glu Arg Thr Met Arg Gln Arg Asp Arg Glu Ala Gly Arg Gly Leu

        115                 120                 125

gga cgc atg cgc cgg ggg ctg ctc tat gac agc agc gag gaa gat gag 432
Gly Arg Met Arg Arg Gly Leu Leu Tyr Asp Ser Ser Glu Glu Asp Glu
    130                 135                 140

gag cgg cct gcc cgt aag cgc cgc cac gta gaa cgc gcc aca gag gat 480
Glu Arg Pro Ala Arg Lys Arg Arg His Val Glu Arg Ala Thr Glu Asp
145                 150                 155                 160

ggc gag gag gat gaa gag atg atc gag agt att gag aat ctg gag gac 528
Gly Glu Glu Asp Glu Glu Met Ile Glu Ser Ile Glu Asn Leu Glu Asp
                165                 170                 175

ctc aag ggc cac tcg gtg cgc gag cgg gtg agc atg gca ggg ccc agg 576
Leu Lys Gly His Ser Val Arg Glu Arg Val Ser Met Ala Gly Pro Arg
            180                 185                 190

ctg gag atc cac cac cgc ttc aag aac ttc ctg cgc acc cac gtg gac 624
Leu Glu Ile His His Arg Phe Lys Asn Phe Leu Arg Thr His Val Asp
        195                 200                 205

agc cat ggc cac aac gtc ttc aag gag cgc atc agt gat atg tgc aaa 672
Ser His Gly His Asn Val Phe Lys Glu Arg Ile Ser Asp Met Cys Lys
    210                 215                 220

gag aac cgt gag agt ttg gtg gta aat tat gaa gac ctg gca gcc cgg 720
Glu Asn Arg Glu Ser Leu Val Val Asn Tyr Glu Asp Leu Ala Ala Arg
225                 230                 235                 240

gag cac gtg ttg gca tac ttc ctg ccg gaa gca ccg gct gag ttg ctg 768
Glu His Val Leu Ala Tyr Phe Leu Pro Glu Ala Pro Ala Glu Leu Leu
                245                 250                 255

cag atc ttt gac gag gct gcc ctg gag gtc gtg ttg gcc atg tac cct 816
Gln Ile Phe Asp Glu Ala Ala Leu Glu Val Val Leu Ala Met Tyr Pro
            260                 265                 270

aaa tat gac cgt atc acc aac cac atc cat gtg cgc atc tcc cac ctg 864
Lys Tyr Asp Arg Ile Thr Asn His Ile His Val Arg Ile Ser His Leu
        275                 280                 285

cct ctg gtg gag gag ctg cgt tca ctg agg cag ttg cac ctg aac cag 912
Pro Leu Val Glu Glu Leu Arg Ser Leu Arg Gln Leu His Leu Asn Gln
    290                 295                 300

ctg atc cgt acc agt ggc gtg gtg acc agc tgc acc gga gtc ctg ccc 960
Leu Ile Arg Thr Ser Gly Val Val Thr Ser Cys Thr Gly Val Leu Pro
305                 310                 315                 320

cag ctc agc atg gtc aag tac aac tgt agc aag tgc aac ttt gta ctg 1008
Gln Leu Ser Met Val Lys Tyr Asn Cys Ser Lys Cys Asn Phe Val Leu

                325                 330                 335

ggg cct ttc tgc cag tct cag aat cag gag gtg aag cct ggc tcc tgc 1056
Gly Pro Phe Cys Gln Ser Gln Asn Gln Glu Val Lys Pro Gly Ser Cys
            340                 345                 350

cct gag tgc cag tct gct ggg ccc ttt gag atc aac atg gag gag acc 1104
Pro Glu Cys Gln Ser Ala Gly Pro Phe Glu Ile Asn Met Glu Glu Thr
        355                 360                 365

atc tat cag aac tac caa cgt atc cgc atc cag gag agt ccc ggc aag 1152
Ile Tyr Gln Asn Tyr Gln Arg Ile Arg Ile Gln Glu Ser Pro Gly Lys
    370                 375                 380

gtg gcg gct ggc cga ctg ccc cgt tcc aag gat gcc att ctc ctc gct 1200
Val Ala Ala Gly Arg Leu Pro Arg Ser Lys Asp Ala Ile Leu Leu Ala
385                 390                 395                 400

gat ctg gtg gac agc tgc aag cca ggg gac gag att gag ctg acc ggc 1248
Asp Leu Val Asp Ser Cys Lys Pro Gly Asp Glu Ile Glu Leu Thr Gly
                405                 410                 415

att tac cat aat aac tat gac ggc tcg ctt aac acc gcc aac ggc ttt 1296
Ile Tyr His Asn Asn Tyr Asp Gly Ser Leu Asn Thr Ala Asn Gly Phe
            420                 425                 430

cca gtc ttt gcc act att atc ttg gcc aac cat gtt gcc aag aag gac 1344
Pro Val Phe Ala Thr Ile Ile Leu Ala Asn His Val Ala Lys Lys Asp
        435                 440                 445

aac aaa gta gct gtg ggg gag ctc acc gat gag gac gtg aag atg atc 1392
Asn Lys Val Ala Val Gly Glu Leu Thr Asp Glu Asp Val Lys Met Ile
    450                 455                 460

acc ggt ctc tcc aag gat cag caa att gga gag aag atc ttt gcc agc 1440
Thr Gly Leu Ser Lys Asp Gln Gln Ile Gly Glu Lys Ile Phe Ala Ser
465                 470                 475                 480

att gca ccc tcc atc tat ggg cat gaa gac atc aag aga ggc ctg gct 1488
Ile Ala Pro Ser Ile Tyr Gly His Glu Asp Ile Lys Arg Gly Leu Ala
                485                 490                 495

ctg gcc ctg ttt gga ggg gag ccc aag aac cca ggt gga aag cac aag 1536
Leu Ala Leu Phe Gly Gly Glu Pro Lys Asn Pro Gly Gly Lys His Lys
            500                 505                 510

gtt cga ggt gac att aat gtg ctc ttg tgt ggg gac cct ggc aca gca 1584
Val Arg Gly Asp Ile Asn Val Leu Leu Cys Gly Asp Pro Gly Thr Ala
        515                 520                 525

aag tcc caa ttc ctc aaa tac atc gag aaa gtg tct agc cgt gcc atc 1632
Lys Ser Gln Phe Leu Lys Tyr Ile Glu Lys Val Ser Ser Arg Ala Ile

    530                 535                 540

ttc acc act ggc cag ggt gcg tca gca gtg ggt ctc acc gcg tac gtt 1680
Phe Thr Thr Gly Gln Gly Ala Ser Ala Val Gly Leu Thr Ala Tyr Val
545                 550                 555                 560

cag cgg cat ccc gtc agc aga gag tgg acc tta gag gcg gga gcc ctg 1728
Gln Arg His Pro Val Ser Arg Glu Trp Thr Leu Glu Ala Gly Ala Leu
                565                 570                 575

gtt ctg gct gac cgg ggg gtg tgt ctc att gac gag ttt gac aag atg 1776
Val Leu Ala Asp Arg Gly Val Cys Leu Ile Asp Glu Phe Asp Lys Met
            580                 585                 590

aat gac cag gac agg acc agc atc cac gag gcc atg gaa cag caa agc 1824
Asn Asp Gln Asp Arg Thr Ser Ile His Glu Ala Met Glu Gln Gln Ser
        595                 600                 605

atc tcc atc tcc aag gct ggc atc gtt acc tcg ctg caa gcc cgc tgc 1872
Ile Ser Ile Ser Lys Ala Gly Ile Val Thr Ser Leu Gln Ala Arg Cys
    610                 615                 620

act gtc ata gct gct gcc aac ccc ata gga ggc cgc tac gac cct tca 1920
Thr Val Ile Ala Ala Ala Asn Pro Ile Gly Gly Arg Tyr Asp Pro Ser
625                 630                 635                 640

ctg acc ttc tca gag aat gta gac ctc aca gag ccc atc att tcc cgc 1968
Leu Thr Phe Ser Glu Asn Val Asp Leu Thr Glu Pro Ile Ile Ser Arg
                645                 650                 655

ttt gat gtc ctg tgt gtg gtg agg gac act gtt gat cca gtt cag gat 2016
Phe Asp Val Leu Cys Val Val Arg Asp Thr Val Asp Pro Val Gln Asp
            660                 665                 670

gag atg ctg gcc cgc ttt gtg gtt ggc agc cac gtc aga cac cac ccc 2064
Glu Met Leu Ala Arg Phe Val Val Gly Ser His Val Arg His His Pro
        675                 680                 685

agt aac aag aag gat gaa ggg ttg act aat ggt ggc acc ttg gag cca 2112
Ser Asn Lys Lys Asp Glu Gly Leu Thr Asn Gly Gly Thr Leu Glu Pro
    690                 695                 700

gcc atg ccc aac aca tat ggc gtg gag ccc ctg cct cag gag gtg ctg 2160
Ala Met Pro Asn Thr Tyr Gly Val Glu Pro Leu Pro Gln Glu Val Leu
705                 710                 715                 720

aag aag tat atc atc tat gcc aag gag agg gtc cgc ccg aag ctc aac 2208
Lys Lys Tyr Ile Ile Tyr Ala Lys Glu Arg Val Arg Pro Lys Leu Asn
                725                 730                 735

cag atg gac cag gat aaa gtg gcc agg atg tac agt gac ctg agg aag 2256
Gln Met Asp Gln Asp Lys Val Ala Arg Met Tyr Ser Asp Leu Arg Lys

            740                 745                 750

gag tcc atg gca acg ggc agc att ccc atc acg gtg cgc cac atc gag 2304
Glu Ser Met Ala Thr Gly Ser Ile Pro Ile Thr Val Arg His Ile Glu
        755                 760                 765

tcc atg atc cgc atg gcc gag gcc cat gcc cgc atg cac ctg cgg gac 2352
Ser Met Ile Arg Met Ala Glu Ala His Ala Arg Met His Leu Arg Asp
    770                 775                 780

tac gtg atg gaa gac gat gtc aac atg gcc atc cga gtg atg atg gag 2400
Tyr Val Met Glu Asp Asp Val Asn Met Ala Ile Arg Val Met Met Glu
785                 790                 795                 800

agc ttc att gac acc cag aag ttc agc gtc atg cgg agt atg cgc aag 2448
Ser Phe Ile Asp Thr Gln Lys Phe Ser Val Met Arg Ser Met Arg Lys
                805                 810                 815

act ttt gcc cgg tat ctc tcc ttc cgg cga gat aac aat gat ctg ctg 2496
Thr Phe Ala Arg Tyr Leu Ser Phe Arg Arg Asp Asn Asn Asp Leu Leu
            820                 825                 830

ctc ttc ata ctg aag cag ttg gtg gct gag cag gtg aca tat caa cgc 2544
Leu Phe Ile Leu Lys Gln Leu Val Ala Glu Gln Val Thr Tyr Gln Arg
        835                 840                 845

aac cgc ttt ggg gcc cag cag gac acc att gaa ata cct gag aag gat    2592
Asn Arg Phe Gly Ala Gln Gln Asp Thr Ile Glu Ile Pro Glu Lys Asp
    850                 855                 860

ctg atg gac aag gcc agg cag atc aat att cac aac ctc tct gcc ttc    2640
Leu Met Asp Lys Ala Arg Gln Ile Asn Ile His Asn Leu Ser Ala Phe
865                 870                 875                 880

tac gac agc gac ctc ttc aaa ttc aac aag ttc agc cgt gac ctg aaa    2688
Tyr Asp Ser Asp Leu Phe Lys Phe Asn Lys Phe Ser Arg Asp Leu Lys
                885                 890                 895

cgc aaa ctg atc cta cag cag ttc tga                                2715
Arg Lys Leu Ile Leu Gln Gln Phe
            900                 905

<210> 5
<211> 904
<212> PRT
<213> Mus musculus

<400> 5
Met Ala Glu Ser Ser Glu Ser Leu Ser Ala Ser Ser Pro Ala Arg Gln
 1               5                  10                  15
Arg Arg Arg Ile Ser Asp Pro Leu Thr Ser Ser Pro Gly Arg Ser Ser

            20                  25                  30
Arg Arg Ala Asp Ala Leu Thr Ser Ser Pro Gly Arg Asp Leu Pro Pro
        35                  40                  45
Phe Glu Asp Glu Ser Glu Gly Leu Leu Gly Thr Glu Gly Pro Met Glu
    50                  55                  60
Glu Glu Glu Asp Gly Glu Glu Leu Ile Gly Asp Gly Met Glu Arg Asp
65                  70                  75                  80
Tyr Arg Pro Ile Pro Glu Leu Asp Val Tyr Glu Ala Glu Gly Leu Ala
                85                  90                  95
Leu Asp Asp Glu Asp Val Glu Glu Leu Thr Ala Ser Gln Arg Glu Ala
            100                 105                 110
Ala Glu Arg Thr Met Arg Gln Arg Asp Arg Glu Ala Gly Arg Gly Leu
        115                 120                 125
Gly Arg Met Arg Arg Gly Leu Leu Tyr Asp Ser Ser Glu Glu Asp Glu
    130                 135                 140
Glu Arg Pro Ala Arg Lys Arg Arg His Val Glu Arg Ala Thr Glu Asp
145                 150                 155                 160
Gly Glu Glu Asp Glu Glu Met Ile Glu Ser Ile Glu Asn Leu Glu Asp
                165                 170                 175
Leu Lys Gly His Ser Val Arg Glu Arg Val Ser Met Ala Gly Pro Arg
            180                 185                 190
Leu Glu Ile His His Arg Phe Lys Asn Phe Leu Arg Thr His Val Asp
        195                 200                 205
Ser His Gly His Asn Val Phe Lys Glu Arg Ile Ser Asp Met Cys Lys
    210                 215                 220
Glu Asn Arg Glu Ser Leu Val Val Asn Tyr Glu Asp Leu Ala Ala Arg

225                 230                 235                 240

Glu His Val Leu Ala Tyr Phe Leu Pro Glu Ala Pro Ala Glu Leu Leu

                245                 250                 255

Gln Ile Phe Asp Glu Ala Ala Leu Glu Val Val Leu Ala Met Tyr Pro

            260                 265                 270

Lys Tyr Asp Arg Ile Thr Asn His Ile His Val Arg Ile Ser His Leu

        275                 280                 285

Pro Leu Val Glu Glu Leu Arg Ser Leu Arg Gln Leu His Leu Asn Gln

    290                 295                 300

Leu Ile Arg Thr Ser Gly Val Val Thr Ser Cys Thr Gly Val Leu Pro

305                 310                 315                 320

Gln Leu Ser Met Val Lys Tyr Asn Cys Ser Lys Cys Asn Phe Val Leu

                325                 330                 335

Gly Pro Phe Cys Gln Ser Gln Asn Gln Glu Val Lys Pro Gly Ser Cys

            340                 345                 350

Pro Glu Cys Gln Ser Ala Gly Pro Phe Glu Ile Asn Met Glu Glu Thr

        355                 360                 365

Ile Tyr Gln Asn Tyr Gln Arg Ile Arg Ile Gln Glu Ser Pro Gly Lys

    370                 375                 380

Val Ala Ala Gly Arg Leu Pro Arg Ser Lys Asp Ala Ile Leu Leu Ala

385                 390                 395                 400

Asp Leu Val Asp Ser Cys Lys Pro Gly Asp Glu Ile Glu Leu Thr Gly

                405                 410                 415

Ile Tyr His Asn Asn Tyr Asp Gly Ser Leu Asn Thr Ala Asn Gly Phe

            420                 425                 430

Pro Val Phe Ala Thr Ile Ile Leu Ala Asn His Val Ala Lys Lys Asp

        435                 440                 445
Asn Lys Val Ala Val Gly Glu Leu Thr Asp Glu Asp Val Lys Met Ile
    450                 455                 460
Thr Gly Leu Ser Lys Asp Gln Gln Ile Gly Glu Lys Ile Phe Ala Ser
465                 470                 475                 480
Ile Ala Pro Ser Ile Tyr Gly His Glu Asp Ile Lys Arg Gly Leu Ala
                485                 490                 495
Leu Ala Leu Phe Gly Gly Glu Pro Lys Asn Pro Gly Gly Lys His Lys
            500                 505                 510
Val Arg Gly Asp Ile Asn Val Leu Leu Cys Gly Asp Pro Gly Thr Ala
        515                 520                 525
Lys Ser Gln Phe Leu Lys Tyr Ile Glu Lys Val Ser Ser Arg Ala Ile
    530                 535                 540
Phe Thr Thr Gly Gln Gly Ala Ser Ala Val Gly Leu Thr Ala Tyr Val
545                 550                 555                 560
Gln Arg His Pro Val Ser Arg Glu Trp Thr Leu Glu Ala Gly Ala Leu
                565                 570                 575
Val Leu Ala Asp Arg Gly Val Cys Leu Ile Asp Glu Phe Asp Lys Met
            580                 585                 590
Asn Asp Gln Asp Arg Thr Ser Ile His Glu Ala Met Glu Gln Gln Ser
        595                 600                 605
Ile Ser Ile Ser Lys Ala Gly Ile Val Thr Ser Leu Gln Ala Arg Cys
    610                 615                 620
Thr Val Ile Ala Ala Ala Asn Pro Ile Gly Gly Arg Tyr Asp Pro Ser
625                 630                 635                 640
Leu Thr Phe Ser Glu Asn Val Asp Leu Thr Glu Pro Ile Ile Ser Arg

                645                 650                 655
Phe Asp Val Leu Cys Val Val Arg Asp Thr Val Asp Pro Val Gln Asp
            660                 665                 670
Glu Met Leu Ala Arg Phe Val Val Gly Ser His Val Arg His His Pro
        675                 680                 685
Ser Asn Lys Lys Asp Glu Gly Leu Thr Asn Gly Gly Thr Leu Glu Pro
    690                 695                 700
Ala Met Pro Asn Thr Tyr Gly Val Glu Pro Leu Pro Gln Glu Val Leu
705                 710                 715                 720
Lys Lys Tyr Ile Ile Tyr Ala Lys Glu Arg Val Arg Pro Lys Leu Asn
                725                 730                 735
Gln Met Asp Gln Asp Lys Val Ala Arg Met Tyr Ser Asp Leu Arg Lys
            740                 745                 750
Glu Ser Met Ala Thr Gly Ser Ile Pro Ile Thr Val Arg His Ile Glu
        755                 760                 765
Ser Met Ile Arg Met Ala Glu Ala His Ala Arg Met His Leu Arg Asp
    770                 775                 780
Tyr Val Met Glu Asp Asp Val Asn Met Ala Ile Arg Val Met Met Glu
785                 790                 795                 800
Ser Phe Ile Asp Thr Gln Lys Phe Ser Val Met Arg Ser Met Arg Lys
                805                 810                 815
Thr Phe Ala Arg Tyr Leu Ser Phe Arg Arg Asp Asn Asn Asp Leu Leu
            820                 825                 830
Leu Phe Ile Leu Lys Gln Leu Val Ala Glu Gln Val Thr Tyr Gln Arg
        835                 840                 845
Asn Arg Phe Gly Ala Gln Gln Asp Thr Ile Glu Ile Pro Glu Lys Asp

850                    855                    860

Leu Met Asp Lys Ala Arg Gln Ile Asn Ile His Asn Leu Ser Ala Phe

865                    870                    875                    880

Tyr Asp Ser Asp Leu Phe Lys Phe Asn Lys Phe Ser Arg Asp Leu Lys

                    885                    890                    895

Arg Lys Leu Ile Leu Gln Gln Phe

                900

<210> 6

<211> 3187

<212> DNA

<213> Homo sapiens

<220>

<221> CDS

<222> (133)..(1857)

<400> 6

gaattcggca cgagttggag acggcgaccc aggcatctgg ggagcacaga agtcgtactc 60

ccttaaaccc tgctttgctc cccctgtgga tgtaacccct tagctggcat tttgcatctc 120

aattggcttg tg atg gag gcg tct ttg ggg att cag atg gat gag cca atg 171

              Met Glu Ala Ser Leu Gly Ile Gln Met Asp Glu Pro Met

              1               5                   10

gct ttt tct ccc cag cgt gac cgg ttt cag gct gaa ggc tct tta aaa 219
Ala Phe Ser Pro Gln Arg Asp Arg Phe Gln Ala Glu Gly Ser Leu Lys
    15                  20                  25

aaa aac gag cag aat ttt aaa ctt gca ggt gtt aaa aaa gat att gag 267
Lys Asn Glu Gln Asn Phe Lys Leu Ala Gly Val Lys Lys Asp Ile Glu
30                  35                  40                  45

aag ctt tat gaa gct gta cca cag ctt agt aat gtg ttt aag att gag 315
Lys Leu Tyr Glu Ala Val Pro Gln Leu Ser Asn Val Phe Lys Ile Glu
                50                  55                  60

gac aaa att gga gaa ggc act ttc agc tct gtt tat ttg gcc aca gca 363
Asp Lys Ile Gly Glu Gly Thr Phe Ser Ser Val Tyr Leu Ala Thr Ala
            65                  70                  75

cag tta caa gta gga cct gaa gag aaa att gct cta aaa cac ttg att 411
Gln Leu Gln Val Gly Pro Glu Glu Lys Ile Ala Leu Lys His Leu Ile
        80                  85                  90

cca aca agt cat cct ata aga att gca gct gaa ctt cag tgc cta aca 459
Pro Thr Ser His Pro Ile Arg Ile Ala Ala Glu Leu Gln Cys Leu Thr
    95                  100                 105

gtg gct ggg ggg caa gat aat gtc atg gga gtt aaa tac tgc ttt agg 507
Val Ala Gly Gly Gln Asp Asn Val Met Gly Val Lys Tyr Cys Phe Arg

110                 115                 120                 125

aag aat gat cat gta gtt att gct atg cca tat ctg gag cat gag tcg 555
Lys Asn Asp His Val Val Ile Ala Met Pro Tyr Leu Glu His Glu Ser
                130                 135                 140

ttt ttg gac att ctg aat tct ctt tcc ttt caa gaa gta cgg gaa tat 603
Phe Leu Asp Ile Leu Asn Ser Leu Ser Phe Gln Glu Val Arg Glu Tyr
            145                 150                 155

atg ctt aat ctg ttc aaa gct ttg aaa cgc att cat cag ttt ggt att 651
Met Leu Asn Leu Phe Lys Ala Leu Lys Arg Ile His Gln Phe Gly Ile
        160                 165                 170

gtt cac cgt gat gtt aag ccc agc aat ttt tta tat aat agg cgc ctg 699
Val His Arg Asp Val Lys Pro Ser Asn Phe Leu Tyr Asn Arg Arg Leu
    175                 180                 185

aaa aag tat gcc ttg gta gac ttt ggt ttg gcc caa gga acc cat gat 747
Lys Lys Tyr Ala Leu Val Asp Phe Gly Leu Ala Gln Gly Thr His Asp
190                 195                 200                 205

acg aaa ata gag ctt ctt aaa ttt gtc cag tct gaa gct cag cag gaa 795
Thr Lys Ile Glu Leu Leu Lys Phe Val Gln Ser Glu Ala Gln Gln Glu
                210                 215                 220

agg tgt tca caa aac aaa tcc cac ata atc aca gga aac aag att cca 843
Arg Cys Ser Gln Asn Lys Ser His Ile Ile Thr Gly Asn Lys Ile Pro
            225                 230                 235

ctg agt ggc cca gta cct aag gag ctg gat cag cag tcc acc aca aaa 891
Leu Ser Gly Pro Val Pro Lys Glu Leu Asp Gln Gln Ser Thr Thr Lys
        240                 245                 250

gct tct gtt aaa aga ccc tac aca aat gca caa att cag att aaa caa 939
Ala Ser Val Lys Arg Pro Tyr Thr Asn Ala Gln Ile Gln Ile Lys Gln
    255                 260                 265

gga aaa gac gga aag gag gga tct gta ggc ctt tct gtc cag cgc tct 987
Gly Lys Asp Gly Lys Glu Gly Ser Val Gly Leu Ser Val Gln Arg Ser
270                 275                 280                 285

gtt ttt gga gaa aga aat ttc aat ata cac agc tcc att tca cat gag 1035
Val Phe Gly Glu Arg Asn Phe Asn Ile His Ser Ser Ile Ser His Glu
                290                 295                 300

agc cct gca gtg aaa ctc atg aag cag tca aag act gtg gat gta ctg 1083
Ser Pro Ala Val Lys Leu Met Lys Gln Ser Lys Thr Val Asp Val Leu
            305                 310                 315

tct aga aag tta gca aca aaa aag aag gct att tct acg aaa gtt atg 1131
Ser Arg Lys Leu Ala Thr Lys Lys Lys Ala Ile Ser Thr Lys Val Met

        320                 325                 330

aat agt gct gtg atg agg aaa act gcc agt tct tgc cca gct agc ctg 1179
Asn Ser Ala Val Met Arg Lys Thr Ala Ser Ser Cys Pro Ala Ser Leu
    335                 340                 345

acc tgt gac tgc tat gca aca gat aaa gtt tgt agt att tgc ctt tca 1227
Thr Cys Asp Cys Tyr Ala Thr Asp Lys Val Cys Ser Ile Cys Leu Ser
350                 355                 360                 365

agg cgt cag cag gtt gcc cct agg gca ggt aca cca gga ttc aga gca 1275
Arg Arg Gln Gln Val Ala Pro Arg Ala Gly Thr Pro Gly Phe Arg Ala
                370                 375                 380

cca gag gtc ttg aca aag tgc ccc aat caa act aca gca att gac atg 1323
Pro Glu Val Leu Thr Lys Cys Pro Asn Gln Thr Thr Ala Ile Asp Met
            385                 390                 395

tgg tct gca ggt gtc ata ttt ctt tct ttg ctt agt gga cga tat cca 1371
Trp Ser Ala Gly Val Ile Phe Leu Ser Leu Leu Ser Gly Arg Tyr Pro
        400                 405                 410

ttt tat aaa gca agt gat gat tta act gct ttg gcc caa att atg aca 1419
Phe Tyr Lys Ala Ser Asp Asp Leu Thr Ala Leu Ala Gln Ile Met Thr
    415                 420                 425

att agg gga tcc aga gaa act atc caa gct gct aaa act ttt ggg aaa 1467
Ile Arg Gly Ser Arg Glu Thr Ile Gln Ala Ala Lys Thr Phe Gly Lys
430                 435                 440                 445

tca ata tta tgt agc aaa gaa gtt cca gca caa gac ttg aga aaa ctc 1515
Ser Ile Leu Cys Ser Lys Glu Val Pro Ala Gln Asp Leu Arg Lys Leu
                450                 455                 460

tgt gag aga ctc agg ggt atg gat tct agc act ccc aag tta aca agt 1563
Cys Glu Arg Leu Arg Gly Met Asp Ser Ser Thr Pro Lys Leu Thr Ser
            465                 470                 475

gat ata caa ggg cat gct tct cat caa cca gct att tca gag aag act 1611
Asp Ile Gln Gly His Ala Ser His Gln Pro Ala Ile Ser Glu Lys Thr
        480                 485                 490

gac cat aaa gct tct tgc ctc gtt caa aca cct cca gga caa tac tca 1659
Asp His Lys Ala Ser Cys Leu Val Gln Thr Pro Pro Gly Gln Tyr Ser
    495                 500                 505

ggg aat tca ttt aaa aag ggg gat agt aat agc tgt gag cat tgt ttt 1707
Gly Asn Ser Phe Lys Lys Gly Asp Ser Asn Ser Cys Glu His Cys Phe
510                 515                 520                 525

gat gag tat aat acc aat tta gaa ggc tgg aat gag gta cct gat gaa 1755
Asp Glu Tyr Asn Thr Asn Leu Glu Gly Trp Asn Glu Val Pro Asp Glu

                530                 535                 540

gct tat gac ctg ctt gat aaa ctt cta gat cta aat cca gct tca aga 1803
Ala Tyr Asp Leu Leu Asp Lys Leu Leu Asp Leu Asn Pro Ala Ser Arg
            545                 550                 555

ata aca gca gaa gaa gct ttg ttg cat cca ttt ttt aaa gat atg agc 1851
Ile Thr Ala Glu Glu Ala Leu Leu His Pro Phe Phe Lys Asp Met Ser
        560                 565                 570

ttg tga taatggatct tcatttaatg tttactgtta tgaggtagaa taaaaaagaa 1907
Leu
575

tactttgtaa tagccacaag ttcttgttta gagaccagag caggattaat aatttatttt 1967
aacattttag tgtttggtgg cacattctaa aatatagatt aagaatactt aaaatgcctg 2027
ggatagttct tgggactaac aacatgatct tctttgagtt aaacctacct aagtagattt 2087
taggtgggtt cctattaggt cagattttta gcttccctaa ttacctttca ctgacatata 2147
cagaaaaagg agcagtttta gttttaatta attaaaatta acagatgtga tgaggattaa 2207
atgaatcaaa agacttaatt tgtagattct tttagagtta tgagctaggt atagtttggg 2267
gaaactcaac ctggtgctgg tgctcttaac aattttgtaa ataaagaaga taatttcctt 2327
ttctagaggt acatattagg ccttttatga acactaaaac aatgaggaaa tgttggtcat 2387
ggggcaaagt atcacttaaa attgaattca tccattttta aaaaacactt catgaaagca 2447
ttctggtgtg aattgccatt tttttcttac tggcttctca attttcttcc ttctctgccc 2507
ctacctaaaa cattctcctc ggaaattaca tggtgctgac cacaaagttt ctggatgttt 2567
tattaaatat tgtacgtctt tacagttggg aatttaaaat aatacataca ctggttgata 2627

aagggaagct gcaggaccaa ggtgaagatt gatagtccaa atgcttttct tttttgagtt 2687

gtatattttt ggacaccatc ttagatataa ttaggtagct gctgaaagga aaagtgaata 2747

cagaattgac ggtattattg gagatttttc ctctgcgtag agccatccag atctctgtat 2807

cctgttttga ctaagtctta ggtgggttgg gaagacagat aatgaagtgt aggcaaagag 2867

aaaaggaccc aagatagagg tttatattca gaaatggtat atatcaatga cagcatatca 2927

aacttcctat gggaaaaagt ctggtgggtg gtcagctgac agatttccca tttagtagtc 2987

atagaataca gaaatagttt agggacatgt attcattttg ttattttgag cattgatagg 3047

tcagtatatc tacctaatct gtttggtaag tataggatat ataaaccatt accattgatc 3107

tgtcttatgc cataatctta aaaaaaaatt gaatgctctt gaatttgtat attcaataaa 3167

gttatccttt tatatttttt 3187

<210> 7

<211> 574

<212> PRT

<213> Homo sapiens

<400> 7

Met Glu Ala Ser Leu Gly Ile Gln Met Asp Glu Pro Met Ala Phe Ser

1               5                   10                  15

Pro Gln Arg Asp Arg Phe Gln Ala Glu Gly Ser Leu Lys Lys Asn Glu

            20                  25                  30

Gln Asn Phe Lys Leu Ala Gly Val Lys Lys Asp Ile Glu Lys Leu Tyr

        35                  40                  45

Glu Ala Val Pro Gln Leu Ser Asn Val Phe Lys Ile Glu Asp Lys Ile

    50                  55                  60

Gly Glu Gly Thr Phe Ser Ser Val Tyr Leu Ala Thr Ala Gln Leu Gln
65                  70                  75                  80
Val Gly Pro Glu Glu Lys Ile Ala Leu Lys His Leu Ile Pro Thr Ser
                85                  90                  95
His Pro Ile Arg Ile Ala Ala Glu Leu Gln Cys Leu Thr Val Ala Gly
            100                 105                 110
Gly Gln Asp Asn Val Met Gly Val Lys Tyr Cys Phe Arg Lys Asn Asp
        115                 120                 125
His Val Val Ile Ala Met Pro Tyr Leu Glu His Glu Ser Phe Leu Asp
    130                 135                 140
Ile Leu Asn Ser Leu Ser Phe Gln Glu Val Arg Glu Tyr Met Leu Asn
145                 150                 155                 160
Leu Phe Lys Ala Leu Lys Arg Ile His Gln Phe Gly Ile Val His Arg
                165                 170                 175
Asp Val Lys Pro Ser Asn Phe Leu Tyr Asn Arg Arg Leu Lys Lys Tyr
            180                 185                 190
Ala Leu Val Asp Phe Gly Leu Ala Gln Gly Thr His Asp Thr Lys Ile
        195                 200                 205
Glu Leu Leu Lys Phe Val Gln Ser Glu Ala Gln Gln Glu Arg Cys Ser
    210                 215                 220
Gln Asn Lys Ser His Ile Ile Thr Gly Asn Lys Ile Pro Leu Ser Gly
225                 230                 235                 240
Pro Val Pro Lys Glu Leu Asp Gln Gln Ser Thr Thr Lys Ala Ser Val
                245                 250                 255
Lys Arg Pro Tyr Thr Asn Ala Gln Ile Gln Ile Lys Gln Gly Lys Asp
            260                 265                 270

Gly Lys Glu Gly Ser Val Gly Leu Ser Val Gln Arg Ser Val Phe Gly
        275                 280                 285
Glu Arg Asn Phe Asn Ile His Ser Ser Ile Ser His Glu Ser Pro Ala
    290                 295                 300
Val Lys Leu Met Lys Gln Ser Lys Thr Val Asp Val Leu Ser Arg Lys
305                 310                 315                 320
Leu Ala Thr Lys Lys Lys Ala Ile Ser Thr Lys Val Met Asn Ser Ala
                325                 330                 335
Val Met Arg Lys Thr Ala Ser Ser Cys Pro Ala Ser Leu Thr Cys Asp
            340                 345                 350
Cys Tyr Ala Thr Asp Lys Val Cys Ser Ile Cys Leu Ser Arg Arg Gln
        355                 360                 365
Gln Val Ala Pro Arg Ala Gly Thr Pro Gly Phe Arg Ala Pro Glu Val
    370                 375                 380
Leu Thr Lys Cys Pro Asn Gln Thr Thr Ala Ile Asp Met Trp Ser Ala
385                 390                 395                 400
Gly Val Ile Phe Leu Ser Leu Leu Ser Gly Arg Tyr Pro Phe Tyr Lys
                405                 410                 415
Ala Ser Asp Asp Leu Thr Ala Leu Ala Gln Ile Met Thr Ile Arg Gly
            420                 425                 430
Ser Arg Glu Thr Ile Gln Ala Ala Lys Thr Phe Gly Lys Ser Ile Leu
        435                 440                 445
Cys Ser Lys Glu Val Pro Ala Gln Asp Leu Arg Lys Leu Cys Glu Arg
    450                 455                 460
Leu Arg Gly Met Asp Ser Ser Thr Pro Lys Leu Thr Ser Asp Ile Gln
465                 470                 475                 480

Gly His Ala Ser His Gln Pro Ala Ile Ser Glu Lys Thr Asp His Lys
                485                 490                 495
Ala Ser Cys Leu Val Gln Thr Pro Pro Gly Gln Tyr Ser Gly Asn Ser
            500                 505                 510
Phe Lys Lys Gly Asp Ser Asn Ser Cys Glu His Cys Phe Asp Glu Tyr
        515                 520                 525
Asn Thr Asn Leu Glu Gly Trp Asn Glu Val Pro Asp Glu Ala Tyr Asp
    530                 535                 540
Leu Leu Asp Lys Leu Leu Asp Leu Asn Pro Ala Ser Arg Ile Thr Ala
545                 550                 555                 560
Glu Glu Ala Leu Leu His Pro Phe Phe Lys Asp Met Ser Leu
                565                 570

<210> 8
<211> 2780
<212> DNA
<213> Homo sapiens

<220>
<221> CDS
<222> (518)..(2542)

<400> 8
aattcggcac gagctctctg aggctgcgcc aagacctgaa gcggcggacc gagagcccgg 60
gtctgagact gagagagcaa cggaatggag gcggggtaga ggcggaaaca caacctgcag 120

ggccagagcg aggcgcgaga aggacggcgg cgtgaggggg cggggcgcgc agcgcgagaa 180

ggcaggcacg aggggcgagc gcgaggcggg gcacggcgcg tggcgtgaga cggggcgggg 240

cgcgcgtatc ggcgccgcgg ccgcgtgacg cgttttcaaa tcttcaaccg ccgcagccca 300

ctcgtttgtg ctttgcgcct tcctcctccg cgccttggag ccggatccgg ccccggaaac 360

ccgacctgca gacgcggtac ctctactgcg tagaggccgt agctggcgga aggagagagg 420

cggccgtcct gtcaacaggc cgggggaagc cgtgctttcg cggctgcccg gtgcgacact 480

ttctccggac ccagcatgta ggtgccgggc gactgcc atg aac tcc gga gcc atg 535

Met Asn Ser Gly Ala Met

1               5

agg atc cac agt aaa gga cat ttc cag ggt gga atc caa gtc aaa aat 583

Arg Ile His Ser Lys Gly His Phe Gln Gly Gly Ile Gln Val Lys Asn

            10                  15                  20

gaa aaa aac aga cca tct ctg aaa tct ctg aaa act gat aac agg cca 631

Glu Lys Asn Arg Pro Ser Leu Lys Ser Leu Lys Thr Asp Asn Arg Pro

        25                  30                  35

gaa aaa tcc aaa tgt aag cca ctt tgg gga aaa gta ttt tac ctt gac 679

Glu Lys Ser Lys Cys Lys Pro Leu Trp Gly Lys Val Phe Tyr Leu Asp

    40                  45                  50

tta cct tct gtc acc ata tct gaa aaa ctt caa aag gac att aag gat 727

Leu Pro Ser Val Thr Ile Ser Glu Lys Leu Gln Lys Asp Ile Lys Asp

55                  60                  65                  70

ctg gga ggg cga gtt gaa gaa ttt ctc agc aaa gat atc agt tat ctt 775
Leu Gly Gly Arg Val Glu Glu Phe Leu Ser Lys Asp Ile Ser Tyr Leu
                75                  80                  85

att tca aat aag aag gaa gct aaa ttt gca caa acc ttg ggt cga att 823
Ile Ser Asn Lys Lys Glu Ala Lys Phe Ala Gln Thr Leu Gly Arg Ile
            90                  95                  100

tct cct gta cca agt cca gaa tct gca tat act gca gaa acc act tca 871
Ser Pro Val Pro Ser Pro Glu Ser Ala Tyr Thr Ala Glu Thr Thr Ser
        105                 110                 115

cct cat ccc agc cat gat gga agt tca ttt aag tca cca gac aca gtg 919
Pro His Pro Ser His Asp Gly Ser Ser Phe Lys Ser Pro Asp Thr Val
    120                 125                 130

tgt tta agc aga gga aaa tta tta gtt gaa aaa gct atc aag gac cat 967
Cys Leu Ser Arg Gly Lys Leu Leu Val Glu Lys Ala Ile Lys Asp His
135                 140                 145                 150

gat ttt att cct tca aat agt ata tta tca aat gcc ttg tca tgg gga 1015
Asp Phe Ile Pro Ser Asn Ser Ile Leu Ser Asn Ala Leu Ser Trp Gly
                155                 160                 165

gta aaa att ctt cat att gat gac att aga tac tac att gaa caa aag 1063

Val Lys Ile Leu His Ile Asp Asp Ile Arg Tyr Tyr Ile Glu Gln Lys

            170                 175                 180

aaa aaa gag ttg tat tta ctc aag aaa tca agt act tca gta aga gat 1111

Lys Lys Glu Leu Tyr Leu Leu Lys Lys Ser Ser Thr Ser Val Arg Asp

        185                 190                 195

ggg ggc aaa aga gtt ggt agt ggt gca caa aaa aca aga aca gga aga 1159

Gly Gly Lys Arg Val Gly Ser Gly Ala Gln Lys Thr Arg Thr Gly Arg

    200                 205                 210

ctc aaa aag cct ttt gta aag gtg gaa gat atg agc caa ctt tat agg 1207

Leu Lys Lys Pro Phe Val Lys Val Glu Asp Met Ser Gln Leu Tyr Arg

215                 220                 225                 230

cca ttt tat ctt cag ctg acc aat atg cct ttt ata aat tat tct att 1255

Pro Phe Tyr Leu Gln Leu Thr Asn Met Pro Phe Ile Asn Tyr Ser Ile

                235                 240                 245

cag aag ccc tgc agt cca ttt gat gta gac aag cca tct agt atg caa 1303

Gln Lys Pro Cys Ser Pro Phe Asp Val Asp Lys Pro Ser Ser Met Gln

            250                 255                 260

aag caa act cag gtt aaa cta aga atc caa aca gat ggc gat aag tat 1351

Lys Gln Thr Gln Val Lys Leu Arg Ile Gln Thr Asp Gly Asp Lys Tyr

        265                 270                 275

ggt gga acc tca att caa ctc cag ttg aaa gag aag aag aaa aaa gga 1399
Gly Gly Thr Ser Ile Gln Leu Gln Leu Lys Glu Lys Lys Lys Lys Gly
    280                 285                 290

tat tgt gaa tgt tgc ttg cag aaa tat gaa gat cta gaa act cac ctt 1447
Tyr Cys Glu Cys Cys Leu Gln Lys Tyr Glu Asp Leu Glu Thr His Leu
295                 300                 305                 310

cta agt gag caa cac aga aac ttt gca cag agt aac cag tat caa gtt 1495
Leu Ser Glu Gln His Arg Asn Phe Ala Gln Ser Asn Gln Tyr Gln Val
                315                 320                 325

gtt gat gat att gta tct aag tta gtt ttt gac ttt gtg gaa tat gaa 1543
Val Asp Asp Ile Val Ser Lys Leu Val Phe Asp Phe Val Glu Tyr Glu
            330                 335                 340

aag gac aca cct aaa aag aaa aga ata aaa tac agt gtt gga tcc ctt 1591
Lys Asp Thr Pro Lys Lys Lys Arg Ile Lys Tyr Ser Val Gly Ser Leu
        345                 350                 355

tct cct gtt tct gca agt gtc ctg aaa aag act gaa caa aag gaa aaa 1639
Ser Pro Val Ser Ala Ser Val Leu Lys Lys Thr Glu Gln Lys Glu Lys
    360                 365                 370

gtg gaa ttg caa cat att tct cag aaa gat tgc cag gaa gat gat aca 1687

Val Glu Leu Gln His Ile Ser Gln Lys Asp Cys Gln Glu Asp Asp Thr
375                 380                 385                 390

aca gtg aag gag cag aat ttc ctg tat aaa gag acc cag gaa act gaa    1735
Thr Val Lys Glu Gln Asn Phe Leu Tyr Lys Glu Thr Gln Glu Thr Glu
                395                 400                 405

aaa aag ctc ctg ttt att tca gag ccc atc ccc cac cct tca aat gaa    1783
Lys Lys Leu Leu Phe Ile Ser Glu Pro Ile Pro His Pro Ser Asn Glu
            410                 415                 420

ttg aga ggg ctt aat gag aaa atg agt aat aaa tgt tcc atg tta agt    1831
Leu Arg Gly Leu Asn Glu Lys Met Ser Asn Lys Cys Ser Met Leu Ser
        425                 430                 435

aca gct gaa gat gac ata aga cag aat ttt aca cag cta cct cta cat    1879
Thr Ala Glu Asp Asp Ile Arg Gln Asn Phe Thr Gln Leu Pro Leu His
    440                 445                 450

aaa aac aaa cag gaa tgc att ctt gac att tcc gaa cac aca tta agt    1927
Lys Asn Lys Gln Glu Cys Ile Leu Asp Ile Ser Glu His Thr Leu Ser
455                 460                 465                 470

gaa aat gac tta gaa gaa cta agg gta gat cac tat aaa tgt aac ata    1975
Glu Asn Asp Leu Glu Glu Leu Arg Val Asp His Tyr Lys Cys Asn Ile
                475                 480                 485

cag gca tct gta cat gtt tct gat ttc agt aca gat aat agt gga tct 2023
Gln Ala Ser Val His Val Ser Asp Phe Ser Thr Asp Asn Ser Gly Ser
            490                 495                 500

caa cca aaa cag aag tca gat act gtg ctt ttt cca gca aag gat ctc 2071
Gln Pro Lys Gln Lys Ser Asp Thr Val Leu Phe Pro Ala Lys Asp Leu
        505                 510                 515

aag gaa aag gac ctt cat tca ata ttt act cat gat tct ggt ctg ata 2119
Lys Glu Lys Asp Leu His Ser Ile Phe Thr His Asp Ser Gly Leu Ile
    520                 525                 530

aca ata aac agt tca caa gag cac cta act gtt cag gca aag gct cca 2167
Thr Ile Asn Ser Ser Gln Glu His Leu Thr Val Gln Ala Lys Ala Pro
535                 540                 545                 550

ttc cat act cct cct gag gaa ccc aat gaa tgt gac ttc aag aat atg 2215
Phe His Thr Pro Pro Glu Glu Pro Asn Glu Cys Asp Phe Lys Asn Met
                555                 560                 565

gat agt tta cct tct ggt aaa ata cat cga aaa gtg aaa ata ata tta 2263
Asp Ser Leu Pro Ser Gly Lys Ile His Arg Lys Val Lys Ile Ile Leu
            570                 575                 580

gga cga aat aga aaa gaa aat ctg gaa cca aat gct gaa ttt gat aaa 2311

Gly Arg Asn Arg Lys Glu Asn Leu Glu Pro Asn Ala Glu Phe Asp Lys
        585                 590                 595

aga act gaa ttt att aca caa gaa gaa aac aga att tgt agt tca ccg 2359
Arg Thr Glu Phe Ile Thr Gln Glu Glu Asn Arg Ile Cys Ser Ser Pro
    600                 605                 610

gta cag tct tta cta gac ttg ttt cag act agt gaa gag aaa tca gaa 2407
Val Gln Ser Leu Leu Asp Leu Phe Gln Thr Ser Glu Glu Lys Ser Glu
615                 620                 625                 630

ttt ttg ggt ttc aca agc tac aca gaa aag agt ggt ata tgc aat gtt 2455
Phe Leu Gly Phe Thr Ser Tyr Thr Glu Lys Ser Gly Ile Cys Asn Val
                635                 640                 645

tta gat att tgg gaa gag gaa aat tca gat aat ctg tta aca gcg ttt 2503
Leu Asp Ile Trp Glu Glu Glu Asn Ser Asp Asn Leu Leu Thr Ala Phe
            650                 655                 660

ttc tcg tcc cct tca act tct aca ttt act ggc ttt tag aatttaaaa 2552
Phe Ser Ser Pro Ser Thr Ser Thr Phe Thr Gly Phe
665 670 675

atgcatactt ttcagaagtg ataaggatca tattcttgaa atttttataa atatgtatgg 2612
aaattcttag gattttttta ccagctttgt ttacagaccc aaatgtaaat attaaaaata 2672
aatatttgca attttctaca gaattgaata cctgttaaag aaaaattaca gaataaactt 2732

gtgactggtc ttgttttaca ttaaaaaaa aaaaaaaaa aactcgag 2780

<210> 9

<211> 674

<212> PRT

<213> Homo sapiens

<400> 9

Met Asn Ser Gly Ala Met Arg Ile His Ser Lys Gly His Phe Gln Gly
1               5                   10                  15

Gly Ile Gln Val Lys Asn Glu Lys Asn Arg Pro Ser Leu Lys Ser Leu
            20                  25                  30

Lys Thr Asp Asn Arg Pro Glu Lys Ser Lys Cys Lys Pro Leu Trp Gly
        35                  40                  45

Lys Val Phe Tyr Leu Asp Leu Pro Ser Val Thr Ile Ser Glu Lys Leu
    50                  55                  60

Gln Lys Asp Ile Lys Asp Leu Gly Gly Arg Val Glu Glu Phe Leu Ser
65                  70                  75                  80

Lys Asp Ile Ser Tyr Leu Ile Ser Asn Lys Lys Glu Ala Lys Phe Ala
                85                  90                  95

Gln Thr Leu Gly Arg Ile Ser Pro Val Pro Ser Pro Glu Ser Ala Tyr
            100                 105                 110

Thr Ala Glu Thr Thr Ser Pro His Pro Ser His Asp Gly Ser Ser Phe
        115                 120                 125

Lys Ser Pro Asp Thr Val Cys Leu Ser Arg Gly Lys Leu Leu Val Glu

    130                 135                 140
Lys Ala Ile Lys Asp His Asp Phe Ile Pro Ser Asn Ser Ile Leu Ser
145                 150                 155                 160
Asn Ala Leu Ser Trp Gly Val Lys Ile Leu His Ile Asp Asp Ile Arg
                165                 170                 175
Tyr Tyr Ile Glu Gln Lys Lys Lys Glu Leu Tyr Leu Leu Lys Lys Ser
            180                 185                 190
Ser Thr Ser Val Arg Asp Gly Gly Lys Arg Val Gly Ser Gly Ala Gln
        195                 200                 205
Lys Thr Arg Thr Gly Arg Leu Lys Lys Pro Phe Val Lys Val Glu Asp
    210                 215                 220
Met Ser Gln Leu Tyr Arg Pro Phe Tyr Leu Gln Leu Thr Asn Met Pro
225                 230                 235                 240
Phe Ile Asn Tyr Ser Ile Gln Lys Pro Cys Ser Pro Phe Asp Val Asp
                245                 250                 255
Lys Pro Ser Ser Met Gln Lys Gln Thr Gln Val Lys Leu Arg Ile Gln
            260                 265                 270
Thr Asp Gly Asp Lys Tyr Gly Gly Thr Ser Ile Gln Leu Gln Leu Lys
        275                 280                 285
Glu Lys Lys Lys Lys Gly Tyr Cys Glu Cys Cys Leu Gln Lys Tyr Glu
    290                 295                 300
Asp Leu Glu Thr His Leu Leu Ser Glu Gln His Arg Asn Phe Ala Gln
305                 310                 315                 320
Ser Asn Gln Tyr Gln Val Val Asp Asp Ile Val Ser Lys Leu Val Phe
                325                 330                 335
Asp Phe Val Glu Tyr Glu Lys Asp Thr Pro Lys Lys Lys Arg Ile Lys

            340                 345                 350
Tyr Ser Val Gly Ser Leu Ser Pro Val Ser Ala Ser Val Leu Lys Lys
        355                 360                 365
Thr Glu Gln Lys Glu Lys Val Glu Leu Gln His Ile Ser Gln Lys Asp
    370                 375                 380
Cys Gln Glu Asp Asp Thr Thr Val Lys Glu Gln Asn Phe Leu Tyr Lys
385                 390                 395                 400
Glu Thr Gln Glu Thr Glu Lys Lys Leu Leu Phe Ile Ser Glu Pro Ile
                405                 410                 415
Pro His Pro Ser Asn Glu Leu Arg Gly Leu Asn Glu Lys Met Ser Asn
            420                 425                 430
Lys Cys Ser Met Leu Ser Thr Ala Glu Asp Asp Ile Arg Gln Asn Phe
        435                 440                 445
Thr Gln Leu Pro Leu His Lys Asn Lys Gln Glu Cys Ile Leu Asp Ile
    450                 455                 460
Ser Glu His Thr Leu Ser Glu Asn Asp Leu Glu Glu Leu Arg Val Asp
465                 470                 475                 480
His Tyr Lys Cys Asn Ile Gln Ala Ser Val His Val Ser Asp Phe Ser
                485                 490                 495
Thr Asp Asn Ser Gly Ser Gln Pro Lys Gln Lys Ser Asp Thr Val Leu
            500                 505                 510
Phe Pro Ala Lys Asp Leu Lys Glu Lys Asp Leu His Ser Ile Phe Thr
        515                 520                 525
His Asp Ser Gly Leu Ile Thr Ile Asn Ser Ser Gln Glu His Leu Thr
    530                 535                 540
Val Gln Ala Lys Ala Pro Phe His Thr Pro Pro Glu Glu Pro Asn Glu

545                 550                 555                 560

Cys Asp Phe Lys Asn Met Asp Ser Leu Pro Ser Gly Lys Ile His Arg

                    565                 570                 575

Lys Val Lys Ile Ile Leu Gly Arg Asn Arg Lys Glu Asn Leu Glu Pro

                580                 585                 590

Asn Ala Glu Phe Asp Lys Arg Thr Glu Phe Ile Thr Gln Glu Glu Asn

            595                 600                 605

Arg Ile Cys Ser Ser Pro Val Gln Ser Leu Leu Asp Leu Phe Gln Thr

    610                 615                 620

Ser Glu Glu Lys Ser Glu Phe Leu Gly Phe Thr Ser Tyr Thr Glu Lys

625                 630                 635                 640

Ser Gly Ile Cys Asn Val Leu Asp Ile Trp Glu Glu Glu Asn Ser Asp

                    645                 650                 655

Asn Leu Leu Thr Ala Phe Phe Ser Ser Pro Ser Thr Ser Thr Phe Thr

                660                 665                 670

Gly Phe

<210> 10

<211> 176

<212> PRT

<213> Homo sapiens

<400> 10

Asp Ile Arg Tyr Tyr Ile Glu Gln Lys Lys Lys Glu Leu Tyr Leu Leu

1                   5                   10                  15

Lys Lys Ser Ser Thr Ser Val Arg Asp Gly Gly Lys Arg Val Gly Ser

                20                  25                  30

Gly Ala Gln Lys Thr Arg Thr Gly Arg Leu Lys Lys Pro Phe Val Lys

            35                  40                  45

Val Glu Asp Met Ser Gln Leu Tyr Arg Pro Phe Tyr Leu Gln Leu Thr

        50                  55                  60

Asn Met Pro Phe Ile Asn Tyr Ser Ile Gln Lys Pro Cys Ser Pro Phe

65                  70                  75                  80

Asp Val Asp Lys Pro Ser Ser Met Gln Lys Gln Thr Gln Val Lys Leu

                85                  90                  95

Arg Ile Gln Thr Asp Gly Asp Lys Tyr Gly Gly Thr Ser Ile Gln Leu

                100                 105                 110

Gln Leu Lys Glu Lys Lys Lys Lys Gly Tyr Cys Glu Cys Cys Leu Gln

            115                 120                 125

Lys Tyr Glu Asp Leu Glu Thr His Leu Leu Ser Glu Gln His Arg Asn

        130                 135                 140

Phe Ala Gln Ser Asn Gln Tyr Gln Val Val Asp Asp Ile Val Ser Lys
145                 150                 155                 160

Leu Val Phe Asp Phe Val Glu Tyr Glu Lys Asp Thr Pro Lys Lys Lys
                165                 170                 175

<210> 11
<211> 30
<212> DNA
<213> Artificial Sequence

<220>
<223> Description of Artificial Sequence:an artificially
      synthesized primer sequence

<400> 11
cccaagcttg acattagata ctacattgaa                                    30

<210> 12
<211> 30
<212> DNA
<213> Artificial Sequence

<220>

<223> Description of Artificial Sequence:an artificially
synthesized primer sequence

<400> 12
ccggaattct ttctttttag gtgtgtcctt 30

<210> 13
<211> 11
<212> PRT
<213> Artificial Sequence

<220>
<223> Description of Artificial Sequence:TAT sequence

<400> 13
Tyr Gly Arg Lys Lys Arg Arg Gln Arg Arg Arg
1               5                   10

<210> 14
<211> 12
<212> DNA
<213> Artificial Sequence

<220>

<223> Description of Artificial Sequence:an artificially
      synthesized adaptor sequence

<400> 14
aattgcggcc gc                                                          12

<210> 15
<211> 52
<212> DNA
<213> Artificial Sequence

<220>
<223> Description of Artificial Sequence:an artificially
      synthesized primer sequence

<400> 15
ataagaatgc ggccgctaag aaggagatat acatatgtac ccctacgacg tg              52

<210> 16
<211> 37
<212> DNA
<213> Artificial Sequence

<220>

<223> Description of Artificial Sequence:an artificially
      synthesized primer sequence

<400> 16
ataagaatgc ggccgcttat cacaagctca tatcttt                                37

<210> 17
<211> 16
<212> PRT
<213> Artificial Sequence

<220>
<223> Description of Artificial Sequence:an artificially
      synthesized peptide sequence

<400> 17
Met Tyr Pro Tyr Asp Val Pro Asp Tyr Ala Phe Ser Pro Gln Arg Asp
  1               5                  10                  15

<210> 18
<211> 29
<212> DNA
<213> Artificial Sequence

<220>

<223> Description of Artificial Sequence:an artificially

synthesized primer sequence

<400> 18

cacggatcca tggcatccag cccggccca 29

<210> 19

<211> 30

<212> DNA

<213> Artificial Sequence

<220>

<223> Description of Artificial Sequence:an artificially

synthesized primer sequence

<400> 19

gtgctcgagc atcgctgtca tacaggagcc 30

<210> 20

<211> 10

<212> PRT

<213> Artificial Sequence

<220>

<223> Description of Artificial Sequence:an artificially

      synthesized peptide sequence

<220>

<221> MOD_RES

<222> (9)

<223> PHOSPHORYLATION

<400> 20

Cys Arg Gly Asn Asp Pro Leu Thr Ser Ser

  1               5                   10

<210> 21

<211> 10

<212> PRT

<213> Artificial Sequence

<220>

<223> Description of Artificial Sequence:an artificially

      synthesized peptide sequence

<400> 21

Cys Arg Gly Asn Asp Pro Leu Thr Ser Ser

  1               5                   10

## Description

### Technical Field

The present invention relates to methods for measuring the phosphorylation activity of Cdc7-ASK kinase complex.

### Background Art

During cell growth, cells repeat cyclic processes whereby they replicate their genomic DNA, distribute this DNA equally among daughter cells, and then divide. This type of cycle is referred to as a cell cycle. The cell cycle can be divided into the G1 phase (DNA synthesis preparatory phase), S phase (DNA synthesis phase), G2 phase (mitosis preparatory phase), and M phase (mitotic phase). Cells divide by going through each stage in order. The progress of the cell cycle is finely regulated by numerous molecules, controlling unnecessary cell growth. To date, a number of molecules involved in cell cycle progression have been cloned and functionally analyzed. Abnormalities in molecules involved in cell cycle progression have been reported in numerous cancers, and can be considered possible causes of carcinogenesis.

To date, a variety of anti-cancer agents and anticancer agents have been developed. Much abnormal cancer cell growth is recognized as originating from abnormal cell cycle progression. In line with this, anticancer agents that targeting factors which regulate cell cycle progression are being vigorously developed. In particular, the function of Cdk-Cyclin, which has been most researched as a target for anticancer agents, has been considerably analyzed. Numerous candidate molecules for anticancer agents that target Cdk-Cyclin have already been reported.

Cdk is a family of molecules with numerous structural similarities, and each of these molecules forms complexes with various cyclin molecules specific to the cell cycle, thus regulating the various stages of the cell cycle. At present, particular molecules that specifically inhibit only Cdk-Cyclin have not yet been identified from among these structurally similar molecules. In other words, obtaining a molecule that specifically and selectively acts on cancer cells by targeting Cdk-Cyclin has been difficult. In addition, although numerous anticancer agents have been found that target regulatory factors in cell division or the G1 (stationary) phase, few agents have been discovered that target the process of DNA replication, another important regulatory point of cell growth.

A serine/threonine kinase different from Cdk-Cyclin has been found to play an important role at the S phase initiation point (the transition from G1 to S). Namely, in variant line Cdc7, which was isolated as a line with a variant cell division cycle (J. Mol. Biol., 59:183-194, 1971), the Cdc7 protein kinase has been demonstrated to function just prior to the initiation of chromosomal DNA replication, and to be necessary for activation of each origin of replication throughout the S phase (Mol. Cell. Biol., 6:1590-1598, 1986; Genes Dev., 15:480-490, 1998; and Genes Dev., 15:491-501, 1998). In addition, yeast Cdc7 kinase activity is known to be dependent on regulatory subunit, Dbf4 (Genetics, 131:21-29, 1992; and Mol. Cell. Biol., 13:2899-2908, 1993).

Expression of Dbf4 in yeast is cyclical, and regulated at both the transcription and post-translation levels (Exp. Cell Res., 180:419-428, 1989). At least some of the increase in Cdc7 kinase activity during the G1-S transition phase is explained by an increase in expression of Dbf4 during the late G1 phase (Mol. Cell. Biol., 13:2899-2908, 1993; and Exp. Cell Res., 180:419-428, 1989). Moreover, since Dbf4 interacts with the origins of replication in cells (Science, 265:1243-1246, 1994), Cdc7 is thought to trigger S phase initiation by directly activating the replication apparatus formed on an origin of replication.

The present inventors succeeded in isolating H37 protein, which is a human homolog of yeast Dbf4, and DNA encoding it (Unexamined Published Japanese Patent Application No. (JP-A) 2000-135090, J. Biol. Chem., Vol. 275, No. 37, 29042-29052, 2000). H37 is an important regulatory factor that governs the switching on and off of DNA replication initiation. H37 was subsequently named a human Activator of S phase Kinase (ASK).

ASK is a unique gene in human chromosomes, and its function is essential for chromosome replication. Based on the results of developmental engineering genetic analyses using mice, Cdc7 function was verified as being essential at the cellular and animal levels. Cdc7 encodes a kinase catalyst subunit, while ASK encodes each of the active subunits. The two form a complex, comprising an active kinase. The activity of Cdc7-ASK kinase is strictly regulated by the cell cycle, and increases in the S phase when DNA replication occurs. Although this kinase activity is maintained at a high level throughout the S phase, it is no longer detected from the M phase to the G1 phase. Regulation of this activity mainly depends on fluctuations in the level of ASK protein expression. Namely, the amount of ASK protein fluctuates during the cell cycle, and as a result, the activity of human Cdc7 kinase, which is regulated by being bound to ASK, also fluctuates during the cell cycle.

In addition, the expression level and activity of human Cdc7-ASK is typically observed to increase in actively growing cancer cells. In such cancer cells, the activity of human Cdc7 kinase activity is thought to increase with increasing expression of ASK protein. Of even further interest, the present inventors found that the expression level of ASK increases in response to increases in the growth ability of various cultured cancer cells.

Subsequent research by the present inventors demonstrated that the specific substrate phosphorylated by Cdc7-ASK complex is a minichromosome maintenance (MCM) complex (heterohexamer), essential for the initiation of chromosome replication (J. Biol. Chem., Vol. 275, No. 37, 29042-29052, 2000). On the basis of this finding, the present inventors advocate a model in which Cdc7-ASK phosphorylates the MCM present in the replication origin complex, and as a result, induces reconfiguration of the subunit structure, causing the double-stranded DNA split that is essential for replication initiation.

On the basis of this, phosphorylation of a substrate protein by the Cdc7-ASK complex is recognized as being an important phenomenon in regulating the growth of cancer cells. However, many aspects of this phosphorylation mechanism remain to be elucidated.

### Disclosure of the Invention

An object of the present invention is to clarify the mechanism of substrate protein phosphorylation by the Cdc7-ASK complex, and to provide methods for evaluating its activity. In addition, based on these evaluation methods, an object of the present invention is to provide methods for measuring the influence of Cdc7-ASK complex test compounds on the substrate phosphorylation effect. Another object of the present invention is to provide substrates, antibodies, or Cdc7-ASK complexes useful for these methods.

The present inventors considered the Cdc7-ASK complex to be a novel and important anti-cancer agent target; that is, they thought that inhibitors that could inhibit the phosphorylation effect of the Cdc7-ASK complex could selectively regulate cancer cell growth, and would be candidates for effective anticancer agents.

In order to evaluate the action of inhibiting the Cdc7-ASK complex phosphorylation effect, it was first essential to establish methods to evaluate this effect. To achieve this, the present inventors first clarified the mechanism used by the Cdc7-ASK complex to phosphorylate substrate proteins. On the basis of this finding, the present inventors confirmed that the kinase activity of the Cdc7-ASK complex was measurable, thereby completing the present invention.

The present inventors then discovered that the influence of test compounds on the Cdc7-ASK complex's substrate protein phosphorylation function can be evaluated by applying these methods to measure kinase activity. On the basis of this finding, the present inventors established methods for evaluating the inhibitory (or promotional) effect of test compounds on Cdc7-ASK kinase, and methods of screening for compounds comprising such effects, thereby completing the present invention.

Moreover, while establishing these methods, the present inventors discovered novel substrate compounds, antibodies, Cdc7-ASK complexes or so on useful to these methods, thereby completing the present invention. Namely, the present invention relates to methods for measuring the kinase activity of the Cdc7-ASK complex, as below. Alternatively, the present invention relates to methods for evaluating the effect of test compounds on the kinase activity of the Cdc7-ASK complex, and screening methods based on these methods. Moreover, the present invention relates to substrate compounds, antibodies or Cdc7-ASK complexes useful to these methods, or methods for preparing the compounds, antibodies or Cdc7-ASK complexes.
[1] A method for measuring the kinase activity of a Cdc7-ASK complex, comprising the following steps:
   (a) contacting a substrate protein with the Cdc7-ASK complex under conditions that allow phosphorylation of the substrate protein, wherein the substrate protein is a protein comprising the amino acid sequence of SEQ ID NO: 1, or a protein functionally equivalent to that protein;
   (b) measuring the level of phosphorylation of a serine residue of the substrate protein at the position corresponding to position 17 in the amino acid sequence of SEQ ID NO: 1; and
   (c) measuring the kinase activity of the Cdc7-ASK complex using the level of phosphorylation as an indicator.
[2] The method according to [1], wherein the level of phosphorylation is measured based on the level of binding of an antibody that identifies the level of phosphorylation of the serine residue.
[3] The method according to [1], wherein the Cdc7-ASK complex is derived from a biological sample.
[4] A method for measuring the effects of a test compound on the kinase activity of a Cdc7-ASK complex, comprising the following steps:
   (a) contacting a test compound, a substrate protein and a Cdc7-ASK complex active substance, wherein the substrate protein is a protein comprising the amino acid sequence of SEQ ID NO: 1, or a protein functionally equivalent to that protein, and wherein they are contacted in any of the following orders i) to iii):
      i) the test compound and substrate protein are contacted, followed by contacting the Cdc7-ASK complex active substance,
      ii) the substrate protein and Cdc7-ASK complex active substance are contacted in the presence of the test compound, or
      iii) the substrate protein and Cdc7-ASK complex active substance are contacted, followed by contacting the test compound;
   (b) measuring the level of phosphorylation for a serine residue of the substrate protein at the position corresponding to position 17 in the amino acid sequence shown in SEQ ID NO: 1; and
   (c) measuring the effect of the test compound on the kinase activity of the Cdc7-ASK complex active substance using the level of phosphorylation as an indicator.
[5] A method of screening for compounds comprising the effect of regulating the kinase activity of a Cdc7-ASK complex, comprising the following steps:
   (a) measuring the effect of a test compound on the kinase activity of the Cdc7-ASK complex according to the method described in [4]; and
   (b) selecting a test compound with a high or low level of phosphorylation by comparison with a control that has not been contacted with the test compound.
[6] A screening method according to [5], wherein a compound having a low level of phosphorylation is selected in step (b) of [5].
[7] An inhibitor of cell growth comprising a compound selected according to the screening method of [6] as its active ingredient.
[8] A kit for measuring the kinase activity of a Cdc7-ASK complex comprising:
   (a) a substrate protein comprising a continuous amino acid sequence that comprises a serine residue at position 17 of the amino acid sequence of SEQ ID NO: 1, and that which is selected from the amino acid sequence of SEQ ID NO: 1; and
   (b) an antibody that identifies the level of phosphorylation of the serine residue of the substrate protein at the position corresponding to position 17 of the amino acid sequence of SEQ ID NO: 1.
[9] A kit for evaluating the effect of a test compound on the kinase activity of a Cdc7-ASK complex, comprising:
   (a) a Cdc7-ASK complex active substance; and,
   (b) a substrate protein comprising a continuous amino acid sequence that comprises the serine residue at position 17 of the amino acid sequence of SEQ ID NO: 1, and is selected from this amino acid sequence.
[10] A process for producing a Cdc7-ASK complex active substance comprising the following steps:
   (a) introducing a DNA encoding human Cdc7 protein and a DNA encoding a protein comprising the amino acid sequence of SEQ ID NO: 10 or a protein functionally equivalent to the protein, into prokaryotic cells in a state that allows monocistronic expression;
   (b) expressing the two DNAs; and
   (c) recovering the expressed protein.
[11] An antibody that identifies the level of phosphorylation of the serine residue at position 17 of a protein comprising the amino acid sequence of SEQ ID NO: 1.
[12] A protein according to any of the following (a) to (d):
   (a) a protein comprising the amino acid sequence of SEQ ID NO: 1;
   (b) a protein comprising a continuous amino acid sequence that is selected from the amino acid sequence of SEQ ID NO: 3, and comprises the serine of position 17;
   (c) a protein comprising an amino acid sequence in which one or more amino acids in the amino acid sequence of SEQ ID NO: 1 are substituted, deleted, added and/or inserted, wherein the protein is phosphorylated by human Cdc7-ASK complex; and
   (d) a protein comprising an amino acid sequence comprising 90% or more homology with the amino acid sequence of SEQ ID NO: 3, wherein the protein is phosphorylated by human Cdc7-ASK complex.
[13] A protein comprising a continuous amino acid that comprises the amino acid sequence of SEQ ID NO: 10 and that which is selected from the amino acid sequence of SEQ ID NO: 9.
[14] A polypeptide according to [13] that comprises the amino acid sequence of SEQ ID NO: 10.

Alternatively, the present invention relates to methods for inhibiting cell growth that comprise the step of administering a compound selected according to the screening method of [6]. In addition, the present invention relates to the use of a compound, selected according to the screening method of [6], in the production of a cell growth inhibitor.

The present invention relates to methods for measuring the kinase activity of Cdc7-ASK complex, where the methods comprise the following steps:
(a) contacting a substrate protein with the Cdc7-ASK complex under conditions that allow phosphorylation of the substrate protein, wherein the substrate protein is a protein comprising the amino acid sequence of SEQ ID NO: 1, or a protein functionally equivalent to that protein;
(b) measuring the level of phosphorylation of a serine residue of the substrate protein at the position corresponding to position 17 in the amino acid sequence of SEQ ID NO: 1; and
(c) measuring the kinase activity of the Cdc7-ASK complex using the level of phosphorylation as an indicator.

In the present invention, a protein comprising the amino acid sequence of SEQ ID NO: 1, or a protein functionally equivalent to that protein, is used as a substrate protein.

The structure of MCM2 protein is known. The amino acid sequence of human MCM2, and the nucleotide sequence of the cDNA that encodes it, are shown in SEQ ID NOs: 2 and 3 respectively. Cdc7-ASK complex phosphorylates a specific amino acid residue of the human MCM2 of SEQ ID NO: 3. The present inventors have previously demonstrated that Cdc7-ASK complex phosphorylates MCM complex or free MCM2. However, that the Cdc7-ASK complex specifically phosphorylates the serine at position 17 from the MCM2 N-terminal is a novel finding, revealed by the present inventors.

Thus, proteins comprising continuous amino acid sequences that comprise the position 17 serine and are selected from the amino acid sequence of SEQ ID NO: 3, can be used as substrate proteins in the present invention, as long as they can be phosphorylated by Cdc7-ASK complex. Examples of other substrate proteins that can be used in the present invention are indicated below. Tags can be added to these substrate proteins. In addition, these substrate proteins can be bound to a solid phase, as described later.
(a) a protein comprising the amino acid sequence of SEQ ID NO: 1;
(b) a protein comprising a continuous amino acid sequence that is selected from the amino acid sequence of SEQ ID NO: 3, and comprises the serine of position 17;
(c) a protein comprising an amino acid sequence in which one or more amino acids in the amino acid sequence of SEQ ID NO: 1 are substituted, deleted, added and/or inserted, wherein the protein is phosphorylated by human Cdc7-ASK complex; and
(d) a protein comprising an amino acid sequence comprising 90% or more homology with the amino acid sequence of SEQ ID NO: 3, wherein the protein is phosphorylated by human Cdc7-ASK complex.

In the present invention, the phosphorylation of a certain protein by the Cdc7-ASK complex can be confirmed based on the results of, for example, the reactions described in Examples. Namely, a phosphate compound and the Cdc7-ASK complex are incubated with a substrate protein under conditions that allow phosphorylation of that protein (described below). Next, the level of phosphorylation at the serine residue at the position corresponding to position 17 in the amino acid sequence shown in SEQ ID NO: 1 is measured. When there is no significant difference observed between the measured level of phosphorylation and the level when, for example, a protein comprising the amino acid sequence of SEQ ID NO: 1 is used as a substrate protein, then that protein is judged to be a protein phosphorylated by human Cdc7-ASK complex.

The number of amino acids that make up the substrate protein of the present invention can generally be five or more, usually ten or more, and preferably 50 or more. In addition, although there are no restrictions as to the size of the substrate protein, it can be a short fragment of, for example, 400, generally 300, or 200 or less. Phosphorylation by the Cdc7-ASK complex can be observed more specifically using a short fragment as a substrate protein.

In the present invention, proteins comprising, for example, the amino acid sequence of SEQ ID NO: 1, can be indicated as preferable substrate proteins. The amino acid sequence of SEQ ID NO: 1 is a fragment sequence comprising 130 amino acids on the N-terminal side of the human MCM2 of SEQ ID NO: 3. The serine located at the 17th amino acid counting from the N-terminal is phosphorylated by the Cdc7-ASK complex.

In addition, proteins which are phosphorylated by human Cdc7-ASK complex and that comprise amino acid sequences in which one or more amino acids in the amino acid sequence of SEQ ID NO: 1 are substituted, deleted, added and/or inserted, can also be used as substrate proteins. In the present invention the number of amino acids subjected to mutation is, for example, one to ten, preferably one to six, and more preferably one to three.

Mutation of an amino acid sequence may be a mutation that is induced artificially, or that occurs spontaneously. When substituting amino acids, conservative substitution can be used. In general, to maintain protein function, the substitute amino acids are preferably those with properties similar to the amino acids prior to substitution. This type of amino acid substitution is referred to as conservative substitution.

For example, since Ala, Val, Leu, Ile, Pro, Met, Phe and Trp are all classified as non-polar amino acids, they have mutually similar properties. Examples of non-charged amino acids include Gly, Ser, Thr, Cys, Tyr, Asn and Gln. Alternatively, examples of acidic amino acids include Asp and Glu. Moreover, examples of basic amino acids include Lys, Arg and His. The amino acids that compose each of these groups have mutually similar properties. Consequently, there is a high possibility that protein function will be maintained when these amino acids are substituted with other amino acids from the same group.

This type of protein can be obtained by introducing a mutation into the nucleotide sequence of the cDNA of the human MCM2 of SEQ ID NO: 1. Technology for introducing mutations into genes comprising known nucleotide sequences is known. Alternatively, proteins comprising desired amino acid sequences can be prepared by chemical synthesis.

In the substrate proteins of the present invention, the serine located at the position corresponding to position 17 of the amino acid sequence of SEQ ID NO: 1 is important as the phosphorylation target of the Cdc7-ASK complex. Thus, even when the substrate protein is a protein comprising an amino acid sequence mutation, it is important that a serine be preserved at position 17, or a position homologous to position 17. The term "position homologous to position 17" refers to a position that corresponds to position 17 of the amino acid sequence of SEQ ID NO: 1, when a given amino acid sequence is aligned with the amino acid sequence of SEQ ID NO: 1. Multiple methods are known for aligning amino acid sequences. For example, a wide variety of software, based on algorithms such as the BLAST algorithm, is in practical use for aligning different amino acid sequences.

In addition, a protein that comprises an amino acid sequence comprising 90% or more homology to the amino acid sequence of SEQ ID NO: 3, and which is phosphorylated by human Cdc7-ASK complex, can also be used as a substrate protein of the present invention. For example, human Cdc7-ASK complex phosphorylates not only human MCM2, but also mouse MCM2. Thus, mouse MCM2 can be used as a substrate protein in the present invention. The amino acid sequence of mouse MCM2 is shown in SEQ ID NO: 5, while the nucleotide sequence that encodes this amino acid sequence is shown in SEQ ID NO: 4. Mouse MCM2 and human MCMC2 are 99% homologous. Thus, proteins comprising 99% or more homology to the amino acid sequence of human SEQ ID NO: 3 (the full-length amino acid sequence of human MCM2) are useful as substrate proteins of the present invention.

In the present invention, the level of phosphorylation of the serine at position 17 of human MCM2, or of a serine at a position homologous to this serine, is measured as an indicator. When using a protein that comprises an amino acid sequence different from the amino acid sequences of SEQ ID NOs: 1 and 3 as a substrate protein, it is important to preserve the serine at position 17 of SEQ ID NO: 1, or serines at positions homologous to position 17. For example, in mouse MCM2, the serine at position 26 is the serine at a position homologous to position 17. Thus, when using mouse MCM2 fragments as substrate proteins, it is preferable to use proteins that comprise an amino acid sequence comprising a serine at position 26.

The substrate protein of the present invention can be obtained based on genetic engineering techniques. Namely, a protein having the desired amino acid sequence can be obtained by expressing a DNA encoding MCM2 in a suitable expression system. The nucleotide sequence of a DNA encoding MCM2 is shown in SEQ ID NO: 2.

For example, the Examples show an example of expressing the MCM2 gene using *Escherichia coli*. The expression product can be purified using techniques such as salting out, gel filtration, ion exchange chromatography or so on. Purification by affinity chromatography, using the binding affinity of a tag with binding affinity that has been fused to MCM2, can also be used. Examples of binding tags that can be used include a histidine tag comprising several histidines (His-Tag), β-D-galactosidase, glutathione S-transferase (GST), thioredoxin, maltose-binding protein, Myc, Xpress, and FLAG. If GST is used, for example, the expressed protein can be purified easily with a Glutathione Sepharose 4B column or so on.

Depending on the expression system, MCM2 may be recovered in a phosphorylated state. When the substrate protein has been phosphorylated in the expression system, the protein can be dephosphorylated by treatment with an enzyme such as phosphatase, which acts on phosphate groups. The measurement methods of the present invention comprise the step of measurement using the phosphorylation of substrate proteins as an indicator. Thus, it is particularly preferable that the substrate protein sites to be phosphorylated by the Cdc7-ASK complex are not phosphorylated. In addition, methods are also known for chemically synthesizing proteins comprising the desired amino acid sequence.

The present invention's methods for measuring the kinase activity of Cdc7-ASK complex comprise the step of contacting the aforementioned substrate proteins with samples whose Cdc7-ASK complex kinase activity is to be measured, under conditions that allow phosphorylation of the substrate protein. In the present invention, "conditions that allow phosphorylation of the substrate protein" refer to conditions suited to expression of Cdc7-ASK complex kinase activity. More specifically, the temperature, salt concentration, and pH are suited to the expression of enzyme activity, and phosphate compounds are present for substrate protein phosphorylation. "Conditions suitable for enzyme activity" can be exemplified as below. Namely, such reaction conditions can be imparted by contacting the aforementioned components in HEPES buffer adjusted to pH 7.0 to 7.5.

In the present invention, "conditions that allow phosphorylation of a substrate protein" refer to contacting a sample with a substrate protein in the presence of a compound comprising a phosphate group necessary for substrate protein phosphorylation. Phosphate groups can be supplied by, for example, coexisting with adenosine triphosphate (hereinafter described as ATP).

The substrate protein and phosphate compound that comprise the aforementioned reaction are preferably added in excess with regards to the Cdc7-ASK complex kinase activity in the sample. It is generally difficult to accurately predict the kinase activity of Cdc7-ASK complex contained in a sample. However, those with ordinary skill in the art are able to experimentally predetermine an adequate amount of substrate compound or phosphate compound, such that the components required by the reaction are sufficient for enzyme reaction. For example, when measuring a human-derived biological sample, the amount of substrate protein to be used is, for example, 0.1 µg or more, and generally 0.2 µg to 1 µg. The amount of phosphate compound to be added as ATP can be, for example, 0.01 mM or more, and generally 0.1 mM to 1 mM. Furthermore, phosphate compound is added in an amount that is necessary and sufficient for phosphorylation of the substrate protein used in the reaction system.

The reaction time for phosphorylation is typically ten minutes or more, and preferably 30 minutes or more. Reliable phosphorylation of substrate protein can be expected from, for example, 50 to 100 minutes of reaction. The time required for phosphorylation varies according to the amount of substrate protein used, and the level of Cdc7-ASK complex activity in the reaction solution. Those with ordinary skill in the art are able to suitably set the reaction time required for phosphorylation under given conditions.

In the measurement methods of the present invention, the level of substrate protein phosphorylation over a predetermined time relates to phosphorylation activity in the sample. Alternatively, the phosphorylation activity in a sample can also be measured by using, as an indicator, the reaction time required to phosphorylate a substrate protein to a predetermined level. Namely, the greater a sample's phosphorylation activity, the faster the substrate protein phosphorylation will reach a predetermined level. Moreover, phosphorylation activity in the sample can also be measured by using the amount of sample required to phosphorylate the substrate protein to a predetermined level as an indicator. In this embodiment, the amount of sample required for phosphorylation is reversely proportional to the level of phosphorylation activity in the sample.

When the phosphorylation activity in a sample far exceeds that predicted, the majority of the substrate protein will be phosphorylated in a short period of time. If this happens, accurate evaluation of phosphorylation activity may be impossible. Thus, when such results are obtained, it is preferable to reduce the amount of sample added to the reaction, and remeasure phosphorylation activity.

The measurement methods of the present invention comprise the step of measuring the phosphorylation level of the substrate protein for the serine residue at the position corresponding to position 17 of the amino acid sequence of SEQ ID NO: 1. The level of phosphorylation at this position can be measured immunologically by using, for example, an antibody that recognizes the phosphorylated state of the serine residue at a position corresponding to position 17.

More specifically, an antibody can be used for which binding activity decreases with serine dephosphorylation, as compared to binding activity to an antigen-determining group comprising the phosphorylated serine residue of a protein with an amino acid sequence comprising a serine that corresponds to position 17. Conversely, antibodies that bind to antigen-determining groups comprising a non-phosphorylated serine residue, but for which bonding activity is decreased with phosphorylation of the serine residue, can also be used.

In the measurement methods of the present invention, antibodies that identify the level of phosphorylation of the aforementioned serine residue, used to measure the level of phosphorylation, can be obtained in, for example, the following manner: First, a synthetic peptide that comprises a continuous amino acid sequence comprising the applicable serine residue can be used as the immunogen. The length of the synthetic peptide used as the immunogen can be at least three or more, for example five or more, normally ten or more, and preferably ten to 20 amino acids. A preferable example of an immunogen is an amino acid sequence composed of 15 continuous amino acids, containing serine at position 17, and selected from the amino acid sequence described in SEQ ID NO: 1. The position of the serine can be arbitrary. For example, in the Examples, a synthetic peptide was used as an immunogen that comprised an amino acid sequence in which serine was located in the center of 15 amino acids. The synthetic peptide can be bonded to a carrier protein. Keyhole Limpet hemocyanin and such can be used as the carrier protein.

The serine residue corresponding to position 17 in the immunogen can be phosphorylated in advance. Methods for obtaining synthetic peptides in which a specific amino acid has been phosphorylated are known. When the serine in the immunogen is phosphorylated, antibodies comprising the activity of binding to phosphorylated serine can be obtained.

Immunogens are administered to immunity animals by mixing with suitable adjuvants. Immunization is normally performed several times, and blood is collected from immunized animals after confirming an adequate rise in antibody titer. Serum recovered from the collected blood can be used as antiserum that contains an antibody of the present invention. Purified antibodies can be obtained by purifying immunoglobulins from such antiserums.

Alternatively, cell lines that produce monoclonal antibodies can be established by immortalizing antibody-producing cells from the immunized animals, and then selecting clones that produce antibodies comprising the desired reactivity. For example, antibody-producing cells can be immortalized by fusion with cells such as myeloma cells, to produce hybridomas.

Antibodies can be confirmed to have desired reactivity by using the synthetic peptide used as the immunogen. For example, antibodies that recognize phosphorylated substrate proteins can be obtained by selecting antibodies that bind to synthetic peptides comprising phosphorylated serine. Moreover, an antibody that specifically binds to a phosphorylated substrate protein can be obtained by removing from this antibody any antibodies that bind to synthetic peptides that comprise non-phosphorylated serine. Using this kind of selection process, antibodies that recognize the phosphorylated state of substrate proteins, essential to the present invention, can be obtained by treating with antiserum or purified antibody.

Hybridomas that produce monoclonal antibodies able to recognize the phosphorylated state of substrate proteins can be selected by screening antibodies produced by the hybridomas, according to similar selection processes.

In the measurement methods of the present invention, the aforementioned antibodies can be labeled in advance. Antibodies bound to phosphorylated sites can be easily detected by labeling antibodies. In addition, if the signals from labels can be amplified, measurements of higher sensitivity can be expected.

Arbitrary labeling components can be used for antibody labeling. For example, fluorescent dyes, enzymes, radioactive substances, and so on can be used as labels. In addition, substances with high electron density, such as ferritin and gold colloids, can also be used as labels.

Examples of fluorescent dyes include fluorescein isothiocyanate (FITC), rhodamine β-isothiocyanate (RITC), and phycoerythrin (PE). Examples of enzymes include peroxidase, β-D-galactosidase, microperoxidase, alkaline phosphatase, acidic phosphatase, and cytochrome c. Examples of radioactive substances include ¹²⁵I, ¹⁴C and ³H. Methods for labeling antibodies with these labeling components are known.

In addition, antibodies can also be labeled using antibodies (hereinafter referred to as secondary antibodies) that bind to phosphorylation-recognizing antibodies (primary antibodies). For example, a phosphorylation-recognizing antibody is contacted with a substrate protein, and then reacted with a secondary antibody. The amount of secondary antibody indirectly bound to the substrate is measured, and the amount of bound primary antibody is then determined from the measured amount of secondary antibody. Thus, the level of substrate phosphorylation can be determined. For example, when using a mouse monoclonal antibody as a primary antibody, anti-mouse IgG goat antibody can be used as a secondary antibody.

Alternatively, a primary antibody can also be indirectly labeled by labeling with a substance with binding affinity, and then using the affinity of a binding partner to that affinitive substance. For example, if a primary antibody is avidinyllated, a biotinylated enzyme can be bound to the antibody using the affinity between avidin and biotin.

In the measurement methods of the present invention, Cdc7-ASK substrate protein can be used in an immobilized state by pre-binding it to an insoluble support. Immobilizing the substrate protein makes it easier to detect binding with the aforementioned phosphorylation-recognizing antibodies. In other words, bound and unbound antibodies are easily separated by contacting the antibodies with a substrate protein, removing the liquid phase, and washing the solid phase. The amount of antibody bound (or not bound) to the substrate protein can then be easily measured by measuring the antibody in the solid phase (or liquid phase). Immobilizing substrate proteins on a solid phase also contributes to their stabilization.

Insoluble supports used for immobilizing substrate proteins include resins such as polystyrene resins, polycarbonate resins, silicon resins and Nylon resins, glass, and micelle particles, and the material used is not particularly restricted. There are also no particular restrictions on the form of the insoluble support, and examples of forms that can be used include trays, spheres, rods, fibers, cells and test tubes. The substrate proteins can be immobilized on insoluble supports by physical adsorption or chemical bonding. The insoluble support on which a substrate protein has been immobilized can also be blocked as necessary. Albumin, skim milk, and so on are used for blocking. Blocking the insoluble support inhibits non-specific antibody binding. In addition, blocking can also be expected to exhibit a protective effect on the substrate protein.

In the present invention, the amount of antibody bound (or not bound) to a substrate protein is correlated with a sample's Cdc7-ASK complex kinase activity. More specifically, when using, for example, an antibody that binds to an antigen-determining group comprising a substrate protein's phosphorylated serine, the amount of antibody bound to the substrate protein is proportional to the degree of kinase activity of the Cdc7-ASK complex in the sample. The kinase activity in a sample can also be quantitatively determined by comparison with the results of measurements obtained from a Cdc7-ASK complex standard, for which the level of kinase activity is already known.

The present invention measures the kinase activity of Cdc7-ASK based on the phosphorylation state of a substrate protein region phosphorylated by Cdc7-ASK. Thus, the kinase activity of Cdc7-ASK can be specifically measured, without being affected by other substances that comprise phosphorylation activity and which may coexist in the sample. As a result, the state of cell growth can be specifically detected by measuring Cdc7-ASK kinase activity based on the methods of the present invention.

Cdc7-ASK maintains a high level of activity, particularly in the S phase of the cell cycle, and its activity decreases in the G1 phase. Thus, measuring the activity of Cdc7-ASK kinase is useful as an indicator of a cell's transition from the G1 phase to the S phase. The S phase is the period when cells proceed to replicate nucleic acids in preparation for cell growth. Thus, for example, finding a large number of cells in the S stage among the cells in a certain biological sample means that cell growth is progressing in that sample. More specifically, if a large number of cells with high-level Cdc7-ASK kinase activity are found in a cancer tissue by using a measurement method of the present invention, this cancer tissue contains many actively growing cells. In other words, it is possible that the cancer is highly malignant.

In addition, the present invention relates to methods for measuring the effect of a test compound on the kinase activity of Cdc7-ASK complex, comprising the following steps:
(a) contacting a test compound, a substrate protein and a Cdc7-ASK complex active substance, wherein the substrate protein is a protein comprising the amino acid sequence of SEQ ID NO: 1, or a protein functionally equivalent to that protein, and wherein they are contacted in any of the following orders i) to iii):
   i) the test compound and substrate protein are contacted, followed by contacting the Cdc7-ASK complex active substance,
   ii) the substrate protein and Cdc7-ASK complex active substance are contacted in the presence of the test compound, or
   iii) the substrate protein and Cdc7-ASK complex active substance are contacted, followed by contacting the test compound;
(b) measuring the level of phosphorylation for a serine residue of the substrate protein at the position corresponding to position 17 in the amino acid sequence shown in SEQ ID NO: 1; and
(c) measuring the effect of the test compound on the kinase activity of the Cdc7-ASK complex active substance using the level of phosphorylation as an indicator.

In the aforementioned method, the term "Cdc7-ASK complex active substance" refers to a complex that is functionally equivalent to the Cdc7-ASK complex. More specifically, a "Cdc7-ASK complex active substance" is defined as a complex comprising the function of phosphorylating a substrate protein serine that is to be phosphorylated. Thus, if it makes up a complex that comprises this activity, the complex can still be used as a Cdc7-ASK complex active substance of the present invention, even if the Cdc7 or ASK comprising the complex comprise structures different to that of human-derived proteins.

Whether a certain protein complex comprises the function of phosphorylating a substrate protein serine to be phosphorylated can be confirmed based on, for example, a method such as that shown in the Examples. Namely, the aforementioned substrate protein and protein complex are incubated under conditions that allow phosphorylation of the substrate protein. If there is no significant difference observed when comparing the substrate protein phosphorylation level after this incubation with the level after incubation with human Cdc7-ASK complex, then that protein complex is judged to be functionally equivalent to Cdc7-ASK complex.

The structures of human-derived Cdc7 and ASK are known. The nucleotide sequence of a cDNA encoding human Cdc7 is shown in SEQ ID NO: 6, while an amino acid sequence of human Cdc7 is shown in SEQ ID NO: 7. In addition, the nucleotide sequence of a cDNA encoding human ASK is shown in SEQ ID NO: 8, while an amino acid sequence of human ASK is shown in SEQ ID NO: 9. With regards to these known Cdc7 and ASK, for example, proteins such as the following can be used for each subunit composing a Cdc7-ASK complex active substance of the present invention: In the present invention, those subunits that differ from naturally-occurring human Cdc7 or human ASK, but are capable of making up Cdc7-ASK complex active substances comprising kinase activity, are respectively referred to as Cdc7 subunits and ASK subunits.

First, proteins that can be used as the Cdc7 subunit are those that compose a complex comprising the effect of phosphorylating a substrate protein by forming a complex with an aforementioned human ASK, wherein they are encoded by the following polynucleotides:
(a) a polynucleotide that comprises a region that encodes the nucleotide sequence of SEQ ID NO: 6;
(b) a polynucleotide that comprises a nucleotide sequence that encodes the amino acid sequence of SEQ ID NO: 7;
(c) a polynucleotide that encodes a protein that comprises an amino acid sequence in which one or more amino acids are substituted, deleted, added and/or inserted in the amino acid sequence of SEQ ID NO: 7, in which the polynucleotide composes a complex having phosphorylation effect on a substrate protein by forming a complex with the aforementioned human ASK; and,
(d) a polynucleotide that hybridizes with a polynucleotide that contains a region that encodes the nucleotide sequence of SEQ ID NO: 6 under stringent conditions, in which the polynucleotide encodes a protein that composes a complex having phosphorylation effect on a substrate protein by forming a complex with the aforementioned human ASK.

In the present invention, a protein encoded by an aforementioned polynucleotide can be confirmed to make up a complex comprising the effect of phosphorylating a substrate protein by forming a complex with an aforementioned human ASK in the following manner: Namely, the aforementioned polynucleotide is first co-expressed in suitable host cells along with a DNA comprising a nucleotide sequence encoding human ASK. The phosphorylation activity of the resulting expression product is then evaluated by, for example, a method such as that described in the Examples, and then compared with Cdc7-ASK phosphorylation activity. As a result, if there is no significant difference observed between the two phosphorylation activities, the Cdc7 subunit making up the complex is judged to be a protein that comprises a complex comprising the effect of phosphorylating a substrate protein by forming a complex with the aforementioned human ASK.

On the other hand, proteins encoded by the following polynucleotides and comprising complexes that comprise the effect of phosphorylating substrate proteins by forming complexes with the aforementioned human Cdc7, can be used as ASK subunits that comprise Cdc7-ASK complex active substances of the present invention:
(a) a polynucleotide that comprises a region that encodes the nucleotide sequence of SEQ ID NO: 8;
(b) a polynucleotide that comprises a nucleotide sequence that encodes the amino acid sequence of SEQ ID NO: 9;
(c) a polynucleotide that encodes a protein that comprises an amino acid sequence in which one or more amino acids are substituted, deleted, added and/or inserted in the amino acid sequence of SEQ ID NO: 9, in which the polynucleotide composes a complex having phosphorylation effect on a substrate protein by forming a complex with the aforementioned human Cdc7; and,
(d) a polynucleotide that hybridizes with a polynucleotide that comprises a region that encodes the nucleotide sequence of SEQ ID NO: 8 under stringent conditions, in which the polynucleotide encodes a protein that composes a complex having phosphorylation effect on a substrate protein by forming a complex with the aforementioned human Cdc7.

In the present invention, proteins encoded by the aforementioned polynucleotides can be confirmed to make up complexes comprising the effect of phosphorylating substrate proteins by forming complexes with the aforementioned human Cdc7 in the following manner: Namely, an aforementioned polynucleotide is first co-expressed in suitable host cells along with a DNA comprising a nucleotide sequence that encodes human Cdc7. The phosphorylation activity of the resulting expression product is then evaluated by, for example, a method such as that described in the Examples, and then compared with Cdc7-ASK phosphorylation activity. As a result, if there is no significant difference observed between the phosphorylation activities of the two, the ASK subunit making up the complex is judged to be a protein that comprises a complex comprising the effect of phosphorylating a substrate protein by forming a complex with the aforementioned human Cdc7.

For example, the present inventors clearly demonstrated that a protein comprising an amino acid sequence corresponding to positions 174 to 349 from the N-terminal in the 674 amino acid sequence that comprises human ASK is a region essential to the formation of a complex with Cdc7 and for expression of kinase activity. An amino acid sequence that comprises this region is shown in SEQ ID NO: 10. Such human ASK protein fragments can be used as ASK subunits that compose the Cdc7-ASK complex active substance of the present invention, as long as they comprise the aforementioned activity.

In the present invention, polynucleotides that encode the aforementioned Cdc7 subunits or ASK subunits can be obtained by PCR or hybridized screening from an arbitrary cDNA library. Human libraries as well as those from mammals other than humans such as mice or rats, or eukaryotic cells such as *Caenorhabditis* and *Schizosaccharomyces,* can be used as the cDNA libraries.

The Cdc7-ASK complex active substances of the present invention can be extracted from cells or can be produced using genetic engineering techniques. Production methods using genetic engineering techniques are preferable since they allow a large amount of protein to be easily obtained. A complex comprising human Cdc7 and ASK expressed in insect cells, for example, can be used as a Cdc7-ASK complex active substance of the present invention.

For example, the present inventors have already succeeded in producing such complexes (J. Biol. Chem., Vol. 275, No. 37, 29042-29052, 2000). More specifically, the Cdc7-ASK complex active substances used in the present invention can be obtained by using insect cells in accordance with, for example, the following steps (a) to (c):
(a) introducing a DNA encoding a human Cdc7 subunit and a DNA encoding human ASK subunit into insect cells;
(b) co-expressing the aforementioned two types of introduced DNA in the aforementioned insect cells; and,
(c) purifying the expressed protein (complex).

Any polynucleotide described in the aforementioned (a) to (e) can be used for a DNA that encodes a human Cdc7 subunit used in the aforementioned step (a). Their forms are not particularly restricted, and include cDNA, genomic DNA and synthetic DNA. For example, DNAs comprising the nucleotide sequence shown in SEQ ID NO: 6 can be used as DNAs that encode a human Cdc7 subunit.

Similarly, any polynucleotide described in the aforementioned (a) to (e) can be used as a DNA that encodes a human ASK subunit used in the aforementioned step (a). For example, a DNA comprising the nucleotide sequence shown in SEQ ID NO: 8 can be used as a DNA that encodes a human ASK protein.

Sf9 cells or Sf21 cells derived from *Spodoptera frugiperda*, or Tn5 cells derived from *Trichoplusiani*, can be used as the insect cells. These cells are also commercially available (Invitrogen or Pharmingen), and can also be obtained from ATCC. DNAs encoding a Cdc7 subunit and a ASK subunit can be introduced into insect cells using a baculovirus. For example, a recombinant virus is produced by homologous recombination by subcloning these DNAs into a transfer vector and simultaneously transfecting insect cells with the resulting plasmid and baculovirus DNA. This recombinant virus infects insect cells and co-expresses the Cdc7 subunit and ASK subunit within the insect cells. In order to increase the amount of protein expressed, the recombinant virus is preferably purified and amplified prior to expression of the protein.

Examples of transfer vectors that can be used include commercially available pVL1392 (Pharmingen), pPAK8 (Clontech), pAcUW51 (Pharmingen), pAcUW31 (Clontech), and pAcAB3 (Pharmingen). Transfer vectors capable of simultaneously subcloning a Cdc7 subunit and an ASK subunit are particularly preferable as transfer vectors. For example, two genes can be subcloned by using a transfer vector having two promoters, such as pAcUW51 (Pharmingen), or a transfer vector having three promoters, such as pAcAB3 (Pharmingen). Alternatively, different transfer vectors that respectively subclone the Cdc7 subunit and ASK subunit can also be used.

In addition, examples of baculovirus DNA that can be used include BaculoGold™ Linearized Baculovirus DNA (Pharmingen) and wild type Baculovirus AcNV DNA (Pharmingen, Invitrogen). Various types of kit are commercially available for expression systems using baculovirus, and any of these kits may be used.

The Cdc7 subunit and ASK subunit genes, which are exogenous genes, can be co-expressed in insect cells using the aforementioned transfer vectors and baculoviruses. The Cdc7-ASK complex active substances used in the present invention can be obtained by culturing transformed cells under conditions that allow expression, and then recovering the expression product. To facilitate recovery, suitable tags can be fused to either Cdc7 or ASK or both.

A Cdc7- and ASK-subunit complex can be also obtained in prokaryotic cells, according to the methods described below. For example, when using *Escherichia coli* as the prokaryotic cells, a Cdc7-ASK kinase complex active substance can be prepared according to the following steps:
(A) introducing a DNA encoding human Cdc7 subunit and a DNA encoding a protein comprising the amino acid sequence described in SEQ ID NO: 10 or a protein functionally equivalent to the protein, into *Escherichia coli* in a state that allows monocistronic expression;
(B) expressing the two DNAs; and
(C) recovering the expressed protein.

According to the present inventors' studies, in an *Escherichia coli* system, inserting and expressing a partial ASK (SEQ ID NO: 10), which is not the full length human ASK protein, eventually enabled acquisition of a Cdc7-ASK complex active substance that comprised kinase activity. In the present invention, a protein functionally equivalent to a protein comprising the amino acid sequence of SEQ ID NO: 10 refers to a protein that can form a complex with a Cdc7 subunit and express kinase activity.

Whether or not a certain protein expresses kinase activity by composing a complex with a Cdc7 subunit can be confirmed as follows: Namely, a DNA that encodes the protein is first co-expressed in suitable host cells along with a DNA comprising a nucleotide sequence that encodes human Cdc7. The phosphorylation activity of the resulting expression product is then evaluated by, for example, a method such as that described in the Examples, and this is then compared with the phosphorylation activity of Cdc7-ASK. As a result, if no significant difference is observed between the two phosphorylation activities, the ASK subunit that makes up the complex is judged to be a protein that makes up a complex comprising the effect of phosphorylating a substrate protein by forming a complex with the aforementioned human Cdc7.

Proteins that retain the aforementioned activity and comprise homology of 90% or more with the amino acid sequence of SEQ ID NO: 10 can be indicated as functionally equivalent proteins. In addition, proteins comprising an amino acid sequence in which one or more amino acids are substituted, deleted, added and/or inserted in the amino acid sequence of SEQ ID NO: 10 that retain the aforementioned activity are included in the functionally equivalent proteins. The number of mutated amino acids is normally 20 or less, for example ten or less, and preferably five or less, for example, one to three. Amino acids can be substituted by conservative substitution. Proteins in which a tag has been fused to the amino acid sequence of SEQ ID NO: 10 are included in the functionally equivalent proteins of the present invention.

Step (A) uses a DNA encoding a human Cdc7 subunit similar to that previously described, and a DNA encoding a fragment sequence of a human ASK subunit (hereinafter abbreviated as "partial ASK"). A protein comprising the amino acid sequence shown in SEQ ID NO: 10, or a protein functionally equivalent to the protein, is used as the protein comprising the fragment sequence. The amino sequence of SEQ ID NO: 10 is a partial sequence corresponding to the amino acid sequence from the 174th to the 349th amino acids of SEQ ID NO: 9. The amino acid sequence described in SEQ ID NO: 11 is encoded by the nucleotide sequence described in SEQ ID NO: 10. The nucleotide sequence described in SEQ ID NO: 10 is a partial nucleotide sequence corresponding to the 1027th to the 1564th nucleotide of the nucleotide sequence of SEQ ID NO: 8. Protein fragments comprising this amino acid sequence are regions necessary for the expression of kinase activity by forming a complex with human Cdc7. Herein, as long as a partial ASK encodes a partial ASK protein, it is not limited to this sequence, and DNAs comprising an arbitrary nucleotide sequence that takes codon degeneracy into account are included. The form of this DNA is also unrestricted, and includes cDNA, genomic DNA and synthetic DNA.

In the present invention, the term "a state that allows monocistronic expression" refers to transcribing a DNA encoding a Cdc7 subunit and a DNA encoding an ASK subunit as a single mRNA molecule, and translating them in to two protein molecules. In order to allow monocistronic expression, these DNAs are arranged so as to enable expression under the regulation of a common transcription regulatory region. They are designed so that a nucleotide sequence such as a terminator, which terminates transcription, is not contained between the two genes. The two genes are preferably arranged in close proximity. However, if the two translation frames are consecutive, the genes will be translated as a single fusion protein, and thus a stop codon is arranged between the two genes.

In addition, arranging a ribosome binding sequence (RBS; Shine-Dalgano sequence) between the two genes is effective in facilitating efficient translation of each of the two mRNA protein-encoding regions. In the Examples, a construct is indicated for monocistronic expression in *Escherichia coli* of human Cdc7 and human partial ASK protein comprising the amino acid sequence of SEQ ID NO: 10. The vectors used to produce the construct are not limited. More specifically, by sequentially cloning, for example, DNAs encoding a Cdc7 subunit and an ASK subunit into the cloning site of a commercially available vector such as pGEX-2T, as indicated in the Examples, a vector that allows their monocistronic expression can be obtained.

A desired Cdc7-ASK complex active substance can be obtained by transforming this vector construct in *Escherichia coli* and recovering the expression product from the culture, according to established methods.

The expression product can be purified by a technique such as salting out, gel filtration, or ion exchange chromatography. In methods for producing a complex that use these genetic engineering techniques, a tag can be fused to either or both of the subunits that comprise the complex. Purification can also be used in which a tag with binding affinity for the subunits is fused, followed by affinity chromatography using a substance with binding affinity for this tag. Examples of binding tags that can be used include histidine tags comprising several histidines (His-Tag), β-D-galactosidase, glutathione S-transferase (GST), thioredoxin, maltose-binding protein, Myc, Xpress, and FLAG. If GST is used, for example, the expressed protein can be purified easily with a Glutathione Sepharose 4B column or such.

A substrate protein for measurement of kinase activity according to the present invention can be used as a substrate protein for use in the measurement methods of the present invention, as previously described. In the present invention, a test compound is contacted with a substrate protein and a Cdc7-ASK complex active substance in any of the orders described in the aforementioned i) to iii).
i) By contacting a test compound with a substrate protein, and then contacting the Cdc7-ASK complex active substance, a kinase-modifying effect can be found in the action of the test compound on the substrate protein. ii) By contacting a substrate protein with a Cdc7-ASK complex active substance in the presence of a test compound, competitive inhibitory activity on the kinase activity of the Cdc7-ASK complex active substance can be evaluated. Furthermore, iii) By contacting a substrate protein and Cdc7-ASK complex active substance, then contacting a test compound, the dephosphorylation effect of the test compound on the phosphorylated substrate protein can be detected.

In the present invention, the level of phosphorylation of a substrate protein can be measured according to methods similar to methods for measuring the kinase activity of the present invention, as previously described. For example, the level of phosphorylation is measured by an antibody that recognizes the phosphorylated state at a phosphorylated site of a substrate protein.

The results of measuring the phosphorylation level are correlated with a test compound's effect on the kinase activity of the Cdc7-ASK complex active substance. For example, when the phosphorylation level is reduced in comparison with a control that has not been contacted with a test compound, the test compound is concluded to comprise the activity of inhibiting phosphorylation. In addition, when the level of phosphorylation increases due to a test compound, the test compound is concluded to comprise the effect of promoting the kinase activity of a Cdc7-ASK complex active substance.

Phosphorylation levels can be compared with a control that has not been contacted with a test compound, as well as with the measurement results of a compound that clearly acts on kinase activity. For example, to discover inhibitory effect on kinase activity, the level of a test compound's inhibitory effect can be also evaluated by comparison with a compound already confirmed to comprise a certain degree of inhibitory effect. Furthermore, effects can be evaluated quantitatively by performing an aforementioned measurement method on a substance whose inhibitory effect on kinase activity has already been determined, and then comparing these results with the measurement results of a test compound.

Screening methods for compounds comprising the effect of regulating Cdc7-ASK complex kinase activity can be performed based on methods for measuring the effect of a test compound on Cdc7-ASK complex kinase activity, according to the present invention. Namely, the present invention relates to methods of screening for compounds comprising the effect of regulating Cdc7-ASK complex kinase activity, comprising the following steps:
(a) measuring the effect of test compounds on Cdc7-ASK complex kinase activity using an aforementioned method; and
(b) selecting test compounds that have a high or low level of phosphorylation on comparison with a control.

In the screening methods of the present invention, phosphorylation level can not only be compared with a control that has not been contacted with a test compound, but also with the measurement results of a compound clearly demonstrating an effect on kinase activity. For example, to discover a kinase activity inhibitory effect, the level of a test compound's inhibitory effect can also be evaluated by comparison with a compound already confirmed to comprise a certain degree of inhibitory effect. Compounds comprising an inhibitory effect above a constant level can then be screened by selecting compounds comprising a comparatively higher level of inhibitory effect.

In the screening methods of the present invention, examples of substances that can be used as test compounds include natural or synthetic proteins, peptides, antibodies, cell extracts from animals, plants or bacteria, culture supernatants, and low molecular weight compounds. These test compounds can be obtained from compound libraries or gene libraries.

When the test compound is a natural ingredient, a single compound that inhibits kinase activity can ultimately be identified by fractionating each test compound according to methods known to those skilled in the art (for example, various types of chromatography), and then detecting those test compounds. Compounds inhibiting or promoting kinase activity and isolated by the screening can be used as agents for regulating Cdc7-ASK complex kinase activity.

Cdc7-ASK complex serves as a key cell growth factor in the living body. Thus, cell growth can be regulated by regulating Cdc7-ASK complex kinase activity. For example, compounds that inhibit Cdc7-ASK complex kinase activity are useful as cell growth inhibitors. More specifically, compounds comprising Cdc7-ASK complex inhibitory effect, and selected according to the screening methods of the present invention, are useful for regulating cells such as cancer cells, whose growth should be inhibited.

The screening methods of the present invention specifically detect phosphorylation of a substrate protein by Cdc7-ASK complex. Thus, the effect on Cdc7-ASK complex of compounds selected by the screening methods of the present invention can be said to be more specific. When using such compounds to regulate cancer, for example, the resulting agents can be expected to be highly selective for proliferating cells, since they act specifically on cells in the growth phase.

These compounds are particularly useful as candidate compounds for cancer therapeutic agents. When using a compound isolated according to the screening methods of the present invention as an agent to adjust kinase activity, that compound can be prepared for use in accordance with known pharmaceutical production methods. For example, the compound can be administered to a patient along with a pharmaceutically acceptable carrier or medium (such as physiological saline, a vegetable oil, suspending agent, surfactant or stabilizer). Administration may be performed transcutaneously, nasally, transtracheally, intramuscularly, intravenously, or orally, according to the properties of the compound. Although the dosage varies according to patient age and body weight, patient condition, administration method, and so on, those with ordinary skill in the art can select a suitable dosage.

In addition, the present invention relates to a kit comprising an antibody capable of recognizing the phosphorylated state of a substrate used in the aforementioned measurement methods or screenings. When used to detect kinase activity, kits of the present invention comprise, for example, substrate proteins, buffers and so on, in addition to the aforementioned antibody. In addition, when used to screen for compounds that inhibit or promote kinase activity, Cdc7-ASK complex active substance is also included. Either the substrate protein or the antibody can be labeled as previously described, while the other can be immobilized.

An enzyme standard and substrate protein standard can be included in the kit in order to test the activity of the Cdc7-ASK complex active substance and the measuring system itself. Other ingredients can be added to these standards and above-mentioned antibodies for the purpose of stabilization or such. For example, BSA at about 1%, or polyoles such as sucrose and fructose at a final concentration of 0.2% to 10% (preferably 1%), can be added to the standards as protein denaturation preventives after freeze-drying.

All prior art references cited herein are incorporated by reference in the present specification.

### Brief Description of the Drawings

Fig. 1 shows the arrangement of motif-M and motif-C in human ASK, budding yeast Dbf4, and fission yeast Him1/Cfp1.
Fig. 2 shows results that confirm the specificity of anti-Mcm2-phospho-S17 polyclonal antibody. Absorbance at 450 nm is plotted on the vertical axis, while antibody concentration (ng/ml) is plotted on the horizontal axis.
Fig. 3 shows the results of measuring the protein phosphorylation activity of Cdc7-ASK complex active substance. Absorbance at 450 nm is plotted on the vertical axis, and the amount of enzyme (µL) is plotted on the horizontal axis.
Fig. 4 shows the results of investigating changes in reactivity when the concentration of ATP added to a phosphorylation reaction solution was changed, in a method for measuring Cdc7-ASK complex activity. The relative activity value (%) based on a reactivity of 100 at 2 mM, is plotted on the vertical axis. The final ATP concentration (mM) in the reaction solution is plotted on the horizontal axis.
Fig. 5 shows the results of investigating changes in reactivity when the phosphorylation reaction time was changed, in a method for measuring the activity of Cdc7-ASK complex. Absorbance at 450 nm is plotted on the vertical axis, while the reaction time (minutes) is plotted on the horizontal axis.
Fig. 6 shows the results of evaluating a method for measuring phosphorylation activity by ELISA using Cdc7-ASK complex (wild type, WT) and inactive Cdc7-ASK complex (KD). Absorbance at 450 nm is plotted on the vertical axis, while the amount of enzyme (µL) is plotted on the horizontal axis.
Fig. 7 shows the results of measuring phosphorylation activity by radiofilter assay and ELISA method. The ELISA results (absorbance at 450 nm) are plotted on the left vertical axis; the radiofilter assay results (radioactivity; cpm) are plotted on the right vertical axis; and the amount of enzyme (µL) is plotted on the horizontal axis.
Fig. 8 shows the results of measuring the effects of known protein phosphorylation inhibitors on Cdc7-ASK complex phosphorylation activity. Relative inhibition value (%) is plotted on the vertical axis, while inhibitor concentration (µM) is plotted on the horizontal axis.

### Best Mode for Carrying Out The Invention

The following provide a more detailed explanation of the present invention based on Examples.

All commonly used techniques described in the Examples were carried out in accordance with the publications below. The methods introduced in the various publications can be used for the experimental methods used in the Examples, without any particular restriction.
J. Sambrook, E. F., Fritsch & T. Maniatis (1989), "Molecular Cloning, a laboratory manual, second edition", Cold Spring Harbor Laboratory Press;
Ed Harlow and David Lane (1988), "Antibodies, a laboratory manual", Cold Spring Harbor Laboratory Press;
S. Oumi, K. Tsujimura & M. Inagaki (1994), "Cellular Engineering Supplement, Experimental Protocol Series, Antipeptide antibody experimental protocols", Shujunsha Co., Ltd.; and
T. Migita, S. Konda, H. Motomoro & T. Hamaoka (1995), "Immunology Experimental Procedures I and II", Nankodo Co., Ltd.

### Example 1. Identification of ASK Activity Domain

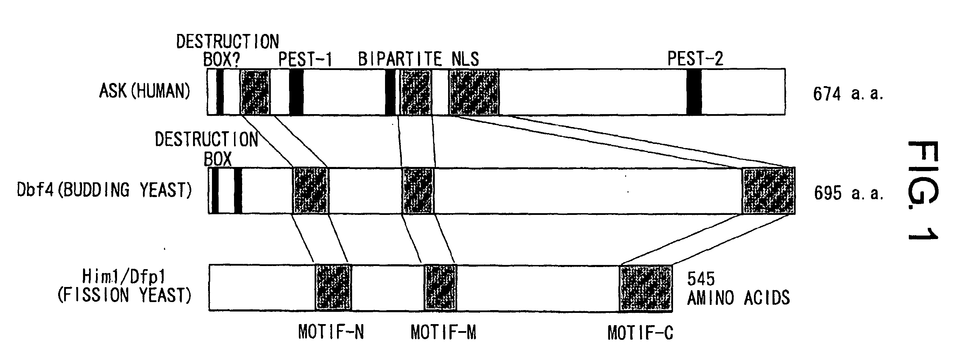

The present inventors conducted detailed mutation analyses using fission yeast Hsk1-Him1/Dfp1 as a material. As a result, the present inventors reported that the presence of only Dbf4-motif-m and Dbf4-motif-C is sufficient for kinase activation (Ogino, K. *et al.*, J. Biol. Chem. 276: 31376-31387, 2001). Fission yeast Hsk1-Him1/Dfp1 is a complex that corresponds to human Cdc7-Dbf4. On the basis of this fact, the present inventors predicted that a minimum domain comprising only motif-M and motif-C would be also be adequate for kinase activation in human ASK. Fig. 1 shows the results of comparing the arrangement of motif-M and motif-C in human ASK, budding yeast Dbf4, and fission yeast Him1/Cfp1.

More specifically, an amino acid sequence corresponding to the 173rd to 349th residue (SEQ ID NO: 10) was selected from the amino acid sequence of human ASK, shown in SEQ ID NO: 9. The DNA encoding this region is precisely divided at introns. The region was selected because introns are advocated to frequently exist at the divisions between functional domains or functional modules.

### Example 2: Vector for Expression of Cdc7-ASK Complex Active Substance

A plasmid designated as GST-TAT-ASK(minimum)-HA-huCdc7 was prepared in order to express the active domains of human Cdc7 and ASK in *E. coli*. First, a GST-TAT-ASK-fused protein expression vector, GST-TAT11, was prepared. A restriction enzyme site (NotI) was then inserted downstream of ASK, and HA-Cdc7 was inserted at this site. At this time, a ribosome binding sequence (RBS; Shine-Dalgano sequence) was added immediately in front of the HA-Cdc7. Due to the addition of this potent RBS, the section from GST-TAT-ASK to HA-Cdc7 was transcribed as one sequence, and both proteins could be efficiently translated from a single transcription product. This detailed procedure is described below:
A region that encodes from the 173rd to the 349th amino acid of the cDNA of human ASK was amplified by PCR using the two primers below:
   Minimum ASK-N (HindIII): CCC AAG CTT GAC ATT AGA TAC TAC ATT GAA (SEQ ID NO: 11); and
   Minimum ASK-C (EcoRI): CCG GAA TTC TTT CTT TTT AGG TGT GTC CTT (SEQ ID NO: 12).

The resulting amplification products were purified, and then cloned into a GST-TAT11 vector. Each region is arranged in this vector in the order of GST-TAT-HindIII-ASK-EcoRI. pGEX-2T-Tat11-TK vector was used as the GST-TAT11 vector. This vector comprises a structure in which a TAT sequence comprising 11 amino acids (YGRKKRRQRRR/SEQ ID NO: 13) is inserted into a GST vector. The HindIII-EcoRI site of TK can be digested to insert minimum ASK, which has been separately amplified with PCR. Since the HindIII frame is AAG CTT, translation in this vector terminates immediately on reaching the stop codon in the 3FRAME, just downstream of the EcoRI site.

Next, the EcoRI site of GST-TAT-HindIII-ASK-EcoRI was changed to a NotI site using an EcoRI-NotI adapter (AATTGCGGCCGC/SEQ ID NO: 14). The full-length coding region was then amplified from the cDNA of human Cdc7, using the primers below:
huCdc7-RBS-N(NotI)/: A TAA GAA TGC GGC CGC TAA Gaa gga gAT ATA CAT atg TAC CCC TAC GAC GTG (SEQ ID NO: 15); and
huCdc7C-NotI: ATAAGAATGCGGCCGCTTATCACAAGCTCATATCTTT (SEQ ID NO: 16).

In the nucleotide sequence of huCdc7-RBS-N(NotI), regions corresponding to RBS and ATG are indicated by lower case letters. The amino acid sequence encoded by the nucleotide sequence starting at ATG is MYPYDVPDYAFSPQRD (SEQ ID NO: 17). The MYPYDVPD of this sequence corresponds to the HA epitope. With this vector, a protein is expressed that comprises an amino acid sequence in which the amino acid sequence starting at the 14th amino acid of human Cdc7 is coupled with the HA tag. As previously mentioned, both genes are translated due to the addition of potent RBS.

Furthermore, wild type and kinase-deactivated type Cdc7 were prepared. The kinase-deactivated type is referred to as K90E, which comprises an amino acid sequence in which the 90th amino acid, lysine (K), is mutated to glutamic acid (E).

### Example 3: Expression and Purification of Cdc7-ASK Complex Active Substance in Bacteria

*E. coli* C600lon- was transformed with the vector GST-TAT-ASK(minimum)-HA-huCdc7, constructed in Example 2 above, in accordance with ordinary methods. C600lon- is a strain of *E. coli* that is missing lon, which is a major *E. coli* protease.

The transformed *E. coli* was inoculated into 200 ml of LB medium containing 40 µg/ml of ampicillin, and cultured at 37°C. When the OD⁶⁰⁰ of the culture medium reached 0.5, IPTG was added at a concentration of 1 mM, and then cultured for an additional three hours. The bacteria were collected by centrifugation, washed, and then suspended in 20 ml of buffer A (40 mM Hepes/KOH (pH 7.6), 1 mM EDTA, 40 mM potassium glutamate, 10% glycerol, and 1 mM DTT). The cells were disrupted using ultrasonic treatment, and the resulting products were fractionated into a soluble fraction and a precipitate (insoluble fraction) by centrifugation. 1 ml of glutathione Sepharose 4B was added to the soluble fraction. After stirring at 4°C for one hour, the glutathione Sepharose 4B was recovered by centrifugation, packed into a simple column, and washed well with buffer A. The column was washed until the OD²⁸⁰ of the eluate reached 0.01 or less, using an amount of buffer A equal to or more than 20 times the column volume. Subsequently, the glutathione Sepharose 4B was eluted first with buffer A containing 20 mM glutathione, and then with buffer A containing 50 mM glutathione.

The fraction finally eluted was separated by SDS-PAGE, and then silver stained, confirming the purification of GST-ASK (about 50 kDa) and HA-Cdc7 (about 65 kDa).

### Example 4: Expression and Purification of MCM2(1-130) by Bacteria

Total RNAs were prepared from Jurkat cells using ISOGEN (Nippon Gene Cat. No. 311-02501), and cDNA was produced using You-Prime First-Strand Beads (Amersham-Pharmacia Cat. No. 27-9264-01). Using this cDNA as a template, human Mcm2(1-130) cDNA was then amplified using the Expand High Fidelity PCR System (Boehringer-Mannheim Cat. No. 1732-650) and the primers below. The resulting 390 bp PCR product was digested with the BamHI and XhoI restriction enzymes, and then cloned into the BamHI/XhoI site of the *E. coli* expression vector, pGEX-4T-1 (Amersham-Pharmacia). The nucleotide sequence was determined, and the sequence of the resulting cDNA was confirmed to be human MCM2(1-130). The primers used for PCR are shown below:
Forward: 5'-CAC GGA TCC ATG GCA TCC AGC CCG GCC CA-3' (SEQ ID NO: 18); and
Reverse: 5'-GTG CTC GAG CAT CGC TGT CAT ACA GGA GCC-3' (SEQ ID NO.: 19).

*E*. *coli* DH5a was transformed with MCM2(1-130)/pGEX-4T-1. This *E. coli* was cultured for about five hours at 30°C, and the culturing temperature was lowered to 20°C when the optical absorbance (600 nm) reached 0.8. IPTG was added to a final concentration of 0.2 mM, and culture was continued for a further 16 hours at 20°C. The collected *E. coli* were suspended in ice-cold solubilizing buffer (PBS, 0.1% TritonX-100 and 1 mM PMSF) and dissolved by disrupting with an ultrasonic crusher on ice. The dissolved crude extract was centrifuged for 30 minutes at 15,000 rpm with a high-speed centrifuge to separate the soluble and insoluble fractions. Each fraction was subjected to SDS-PAGE, and the GST-fusion Mcm2(1-130) was confirmed to be in the soluble fraction. The soluble fraction was added to a column filled with 2 ml of GSH-Sepharose 4B (Amersham-Pharmacia). The column was then washed with 50 ml of washing solution (20 mM Tris-HCl (pH 7.9), 0.5 M NaCl and 5 mM imidazole), and then eluate (10 mM glutathione and 50 mM Tris-HCl (pH 9.6) was used to elute 2 ml into each of ten containers. Optical absorbance (A280 nm) was measured, and that with absorbance of 0.1 or more was dialyzed overnight against ice-cold PBS. Western Blotting using anti-MCM2-specific antibody confirmed that the dialyzed protein was GST-fusion MCM2(1-130).

### Example 5: Identification of Phosphorylated Site of MCM2

First, the primary structures of human, mouse, frog, drosophila, fission yeast, budding yeast, and so on were aligned to identify preserved serine and threonine residues. This was based on the hypothesis that functionally important phosphorylation sites are preserved across species. Eight sites (F1, F2, F3, F4, F5, F6, NF1 and NF2) were selected where these preserved serine and threonine residues formed clusters, being present in relatively large numbers.

Variant MCM2, in which serine and threonine residues 4 to 7 were substituted with alanine or glutamic acid at each site, was prepared for each region. Variants were prepared according to methods that use oligonucleotides. They were then cloned into insect cell expression vector, UW31. UW31 is a vector that can simultaneously express two types of gene products. Recombinant virus solutions co-expressing histidine-tagged MCM7 along with each variant MCM2 were obtained. These were then co-infected into Sf9 insect cells together with a recombinant virus solution that co-expresses histidine-tagged MCM4 and MCM6, and cell extracts were prepared. Affinity purification was then performed on the MCM-2-4-6-7 complex using a nickel column.

The fractions into which the desired MCM-2-4-6-7 complex eluted were pooled. Next, the MCM-2-4-6-7 complex was further purified using a mono-Q column (using the Pharmacia SMART System). The peak fractions eluting at 0.3 to 0.35 M NaCl were pooled. After dialyzing to a lower salt concentration, the fraction was used as a substrate for in vitro phosphorylation reactions.

In addition, and at the same time, polypeptides comprising a portion of these preserved sites were prepared with a histidine or GST tag, expressed in *E. coli*, and then purified. They were prepared by amplifying the intended coding region using a primer oligonucleotide to which a restriction enzyme site had been added, then cloning into a histidine-tagged expression vector (pT7-7/pQE30) or GST-fusion expression vector (pGEX-5X-3). The wild type and an alanine-substituted variant were expressed. These expression vectors were respectively inserted into strain DE3 or C600lon-, and their expression was induced. Affinity purification was then carried out with a nickel column or glutathione Sepharose bead column. These proteins were also used as substrates for *in vitro* phosphorylation reactions.

When the substrate was MCM2-4-6-7 protein complex comprising variant MCM2, phosphorylation was not completely absent regardless of the variant used. Therefore, the phosphorylated wild type and variant MCM2 were digested with trypsin, and then spread out using two-dimensional chromatography to analyze whether or not the spots had disappeared and the phosphorylation sites were restricted.

In addition, as a result of phosphorylation by Cdc7, the SDS-PAGE mobility of MCM2, both *in vivo* and *in vitro*, shifts downward . Using this phenomenon as an indicator, an attempt was made to identify variants without the shift.

From these results, sites phosphorylated by the Cdk and Cdc7 of the MCM2 N-terminal (S27/S41 and S26/S40) were identified. Moreover, this phosphorylation was clearly a cause of the SDS-PAGE shift. The serine residue at position 17 of human MCM2 (S17), which corresponds to the serine residue at position 26 of mouse MCM2 (S26), was demonstrated to be efficiently phosphorylated by Cdc7 when a polypeptide of 130 amino acids from the N-terminal was used as a substrate. On the basis of these results, a protein comprising 130 amino acids from the N-terminal of human MCM2 (SEQ ID NO: 1) was used as a substrate protein for an ELISA assay, described below.

### Example 6: Production of Anti-Phosphorylated Peptide Antibody

Since it is clear that the Cdc7-ASK complex specifically phosphorylates the 17th serine residue of human Mcm2, an anti-phosphorylation-specific antibody that specifically recognizes the phosphorylated 17th serine residue was next produced, as a tool for detecting phosphorylation of the 17th serine residue.

The following two peptides were produced using a peptide synthesizer, and correspond to the amino acid sequence from the 10th to the 20th residue of the human MCM2 protein, comprising the 17th serine residue found to be the phosphorylated site:
Phosphorylated peptide (written as Mcm2-phospho-S17): CRGNDPLTS(p)S (SEQ ID NO: 20); and
Non-phosphorylated peptide (written as Mcm2-S17): CRGNDPLTSS (SEQ ID NO: 21).

The aforementioned peptide sequences are depicted using the single-letter code in the direction from the amino terminal to the carboxyl terminal. "S(p)" indicates the phosphorylated serine residue. The cysteine (C) residue of the amino terminal was introduced to allow the peptide to covalently bond with a carrier protein. Using HPLC, these peptides were confirmed to have a purity of 95% or more. Phosphorylated peptides were used as immunogens. Phosphorylated peptides and non-phosphorylated peptides were used for measuring antibody titer and purifying antibodies.

Alone, the synthesized short peptide has low antigenicity. Therefore, animals are generally immunized using peptides that have been bound to carrier proteins. Albumin, myoglobin, hemocyanin and so on can be used as carrier proteins, however, hemocyanin (keyhole limpet hemocyanin, hereinafter KLH; CALBIOCHEM) was used this time. The carrier protein was covalently bonded using m-maleimidobenzoyl-N-hydroxysuccinimide ester (MBS; SIGMA), which is an agent that crosslinks phospho-Hs-S83 and KLH, in accordance with the attached protocol, and KLH-Mcm2-phospho-S17 was synthesized.

20 µg/100 µl and 10 µg/100 µl of KLH-Mcm2-phospho-S17 were used to respectively immunize rabbits and mice once. 100 µl of Freund's complete adjuvant (Yatron) was then added. Immunogens were then prepared by emulsification. The rabbits were immunized by subcutaneous injection to the back. Immunization was carried out from three to five times every two weeks. After blood was collected from the auricular vein, serum was recovered. Antibody titer was measured by ELISA using a microtiter plate on which Mcm2-phospho-S17 and Mcm2-S17 were immobilized.

Adequate antibody titer was confirmed, and from the next week, a cycle consisting of blood collection (during the first week), rest (during the next week) and immunization (during the final week) was repeated four times. Blood was collected from the auricular vein in the same manner as for titer confirmation. Approximately 60 to 70 ml of blood was collected each time. In the final blood collection, blood was collected directly from the heart using a catheter.

The collected blood was allowed to stand overnight at 4°C, serum was separated from clot, and the serum in the supernatant was recovered. Ammonium sulfate at a final concentration of 50% was added to the separated and recovered serum. The mixture was stired, and then separated by centrifugation. The minimum amount of PBS was added to the precipitate containing the IgG fraction, completely dissolving it. The solution was then dialyzed against PBS. After completely equilibrating with the PBS, the partially purified antibody fraction was applied to a column, and the antibody was purified.

Mcm2-phospho-S17 synthetic peptide was used to produce the specification column, while Mcm2-S17 synthetic peptide was used to produce the absorption column. 1 mg of synthetic peptide was dissolved in 5 ml of 0.1 M carbonate buffer, and then added to CNBr-activated Sepharose 4B (Pharmacia) equilibrated with 1 mM hydrochloric acid. This was gently mixed overnight at 4°C, and added to a column. The column was washed with 5- to 10-fold PBS, and equilibrated with 1 M Tris-HCl (pH 7.5), to block the active groups remaining on the surface of the Sepharose 4B. After blocking, the column was washed and equilibrated with PBS, and used for antibody purification.

To purify the anti-phosphorylated peptide antibodies, the aforementioned partially purified antibody fraction was passed through a specification column. After washing with PBS/0.1% TritonX-100, the anti-phosphorylated peptide antibodies adhered to the column were eluted with 0.17 M glycine-HCl (pH 2.5). The eluted antibodies were immediately neutralized by addition of a suitable amount of 1 M Tris-HCl (pH 8.0), and dialyzed against PBS to obtain anti-Mcm2-phospho-S17 antibody fraction. This was completely equilibrated with PBS, and then passed through the Mcm2-S17 Sepharose 4B column, an absorption column. Passing through this absorption column removes antibodies that also react with the non-phosphorylated peptides present in the anti-Mcm2-phospho-S17 antibody fraction. Anti-phosphorylated peptide-specific antibodies pass through the column without being adsorbed. The absorption column was washed with PBS/0.1% TritonX-100. Antibodies bound to the absorption column were eluted with 0.17 M glycine-HCl (pH 2.5). Following elution, the column was again equilibrated with PBS/0.1% TritonX-100, and the anti-Mcm2-phospho-S17 antibody fraction was repeatedly passed through the absorption column until the non-phosphorylated peptide antibodies were almost completely absorbed. ELISA using a non-phosphorylated peptide-sensitized plate was used to confirm that the antibodies that react with non-phosphorylated peptides were removed. Specificity to the phosphorylated peptide was ultimately confirmed by ELISA using a phosphorylated peptide-sensitized plate.

The peptide was dissolved in 0.1 M carbonate buffer at a concentration of 1 µg/ml. This was dispensed at 50 µl/well to each well of a 96-well microtiter plate for ELISA. This was then left overnight at 4°C to sensitize. Following sensitization, the peptide solution was removed, a blocking solution (1% BSA, 5% sucrose and 0.1% NaN₃/PBS) was added to each well at 200 µl/well, and the plate was allowed to stand for about one hour at room temperature. The blocking solution was then completely removed, followed by air-drying in a draft chamber (immobilization).

The plates on which phosphorylated peptide (Mcm2-phospho-S17) were immobilized were used to measure each antibody titer, confirm absorption of anti-phosphorylated-serine-residue-specific antibody, and confirm the specificity of the anti-phosphorylated-peptide antibody. In addition, plates on which non-phosphorylated peptide (Mcm2-S17) was immobilized were used to confirm column absorption of non-specific antibodies.

The serum for testing antibody titer was serially diluted in 4-fold increments, starting from a 200-fold dilution using PBS. Purified antibody was serially diluted in 4-fold increments starting from a concentration of 1 µg/ml using PBS. The diluted samples were added to the sensitized plates at 50 µl per well. After addition, the plates were allowed to stand for one hour at room temperature (primary antibody reaction). The reaction solution was then discarded and each well was washed four or more times with PBS. Anti-rabbit immunoglobulin antibody (secondary antibody) labeled with horseradish peroxidase (HRP) was then suitably diluted with PBS and added to each well in 50 µl aliquots. This was then reacted for 30 to 60 minutes at room temperature (secondary antibody reaction). Anti-rabbit IgG (H+L-chain) conjugated with peroxidase (MBL) was used as the secondary antibody. The secondary antibody reaction solution was then discarded and each well was washed four or more times with PBS. Coloring substrate solution (750 µM tetramethylbenzidine (TMB)) was then added to each well in 50 µl aliquots, and color was developed for five to 20 minutes at 30°C (coloring reaction). The coloring reaction was stopped by adding 50 µl aliquots of reaction stopping solution (1.5 N H₃PO₄) to each well. Finally, optical absorbance at 450 nm was measured using a microplate reader.

Anti-Mcm2-phospho-S17 antibody was serially diluted in four-fold increments from 4 µg/ml of antibody solution. Specificity was confirmed by ELISA using the plate on which phosphorylated peptide (Mcm2-phospho-S17) was immobilized, and the plate on which non-phosphorylated peptide (Mcm2-S17) was immobilized.

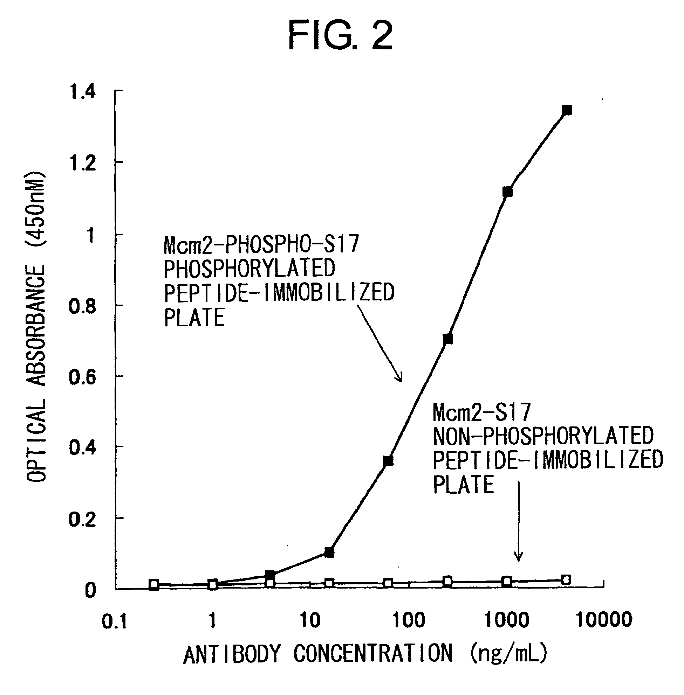

Fig. 2 shows the results of confirming the specificity of anti-Mcm2-phospho-S17 polyclonal antibody. As a result, the optical absorbance of the plate on which Mcm2-phospho-S17 phosphorylated peptide was immobilized increased with increasing antibody concentration. The optical absorbance of the plate on which Mcm2-S17 non-phosphorylated peptide was immobilized remained at nearly zero. Thus, the subject antibody was proven to specifically recognize phosphorylation of the 17th serine residue of human Mcm2.

### Example 7 Construction of an ELISA System for Measuring Cdc7-ASK Complex Phosphorylation Activity

Regions comprising the 17th serine residue of human Mcm2, the substrate used, were selected in order to measure Cdc7-ASK complex phosphorylation activity in a 96-well microtiter plate. More specifically, the substrates studied were 1) GST fusion proteins with the amino acid regions of residues 1 to 130 and 1 to 80 of human MCM2, which phosphorylation assays using radioisotopes found to be a good substrate for Cdc7-ASK complex, and 2) a non-phosphorylated peptide corresponding to the amino acid sequence from the 10th to 20th amino acid residues (Mcm2-S17).

GST-Mcm2 (1-130 a.a.) and GST-Mcm2 (1-80 a.a.) were diluted to 5 µg/ml using 0.1 M carbonate buffer, pipetted at 50 µl/well in to each well of a microtiter plate for ELISA, and then sensitized overnight at 4°C. The sensitizing solution was removed, a blocking solution (1% BSA, 5% sucrose and 0.1% NaN₃/PBS) was pipetted in to each well at 200 µl/well, and the plate was allowed to stand for about one hour at room temperature. The blocking solution was then completely removed. The plates were then air-dried in a draft chamber, and stored at 4°C until use. GST-Mcm2 (1-130 a.a.) and GST-Mcm2 (1-80 a.a.) were immobilized on the inner walls of the microtiter plates by this procedure.

A protein phosphorylation reaction was then carried out in the wells in which recombinant proteins GST-Mcm2 (1-130 a.a.), GST-Mcm2 (1-80 a.a.) or non-phosphorylated peptide (Mcm2-S17) had been immobilized. ELISA was then carried out in succession in the same wells.

A recombinant Cdc7-ASK complex dilution series was prepared by diluting from one-fold (x1) to 64-fold in two-fold increments using a phosphorylation buffer. 50 µl aliquots of this solution were added to the wells of the immobilized plates (a protein phosphorylation reaction). After incubating at 30°C for a fixed period of time, the phosphorylation reaction solution was discarded and each well was amply washed four or more times with PBS. Primary antibody, that is, anti-Mcm2-phospho-S17 polyclonal antibody, was diluted with an antibody diluent (1% BSA, 0.1% NaN₃/PBS) to a concentration of 1 µg/ml, added in 50 µl aliquots to each well, and allowed to react for 30 to 60 minutes at room temperature (primary antibody reaction). The primary antibody reaction solution was then discarded and each well was amply washed four or more times with PBS. Anti-rabbit immunoglobulin antibody (secondary antibody, MBL) labeled with horseradish peroxidase (HRP) was then diluted 1000-fold with PBS and added to each well in 50 µl aliquots, then allowed to react for 30 to 60 minutes at room temperature (secondary antibody reaction). The secondary antibody reaction solution was then discarded and each well was washed four or more times with PBS. A coloring substrate solution was then added to each well in 50 µl aliquots, and color was developed for five to 20 minutes (coloring reaction). The coloring reaction was stopped by adding 50 µl aliquots of reaction stopping solution to each well, and the optical absorbance at 450 nm was measured using a microplate reader.

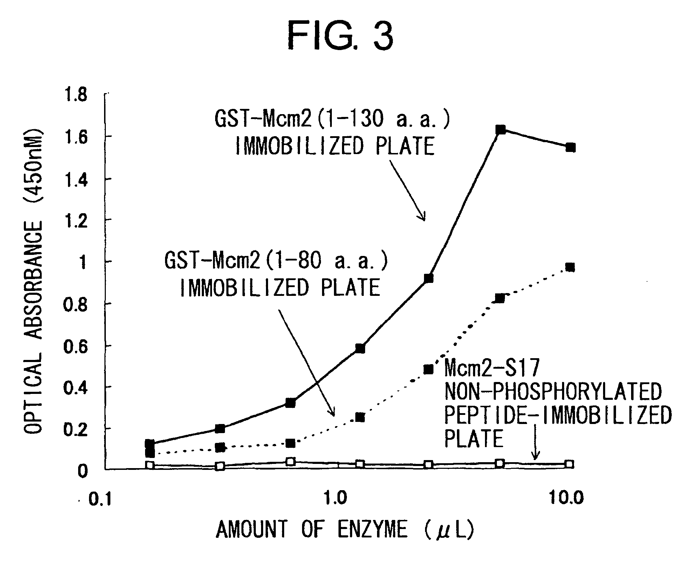

The results are shown in Fig. 3. These results clearly demonstrate that the protein phosphorylation activity of a Cdc7-ASK complex active substance can be measured by ELISA, using the anti-phosphorylated peptide antibodies provided in the present invention. Recombinant protein GST-Mcm2 (1-130 a.a.) was determined to be the most preferable substrate for use in this phosphorylation activity measurement system. One reason why non-phosphorylated peptide (Mcm2-S17) cannot be used in this activity measurement system is presumed to be difficulty in Cdc7-ASK complex recognition of the target 17th serine residue, since the region comprising the phosphorylated site is short. Similar observations also suggest the possibility that GST-Mcm2 (10-20 a.a.) can also be provided as a substrate in the present measurement system.

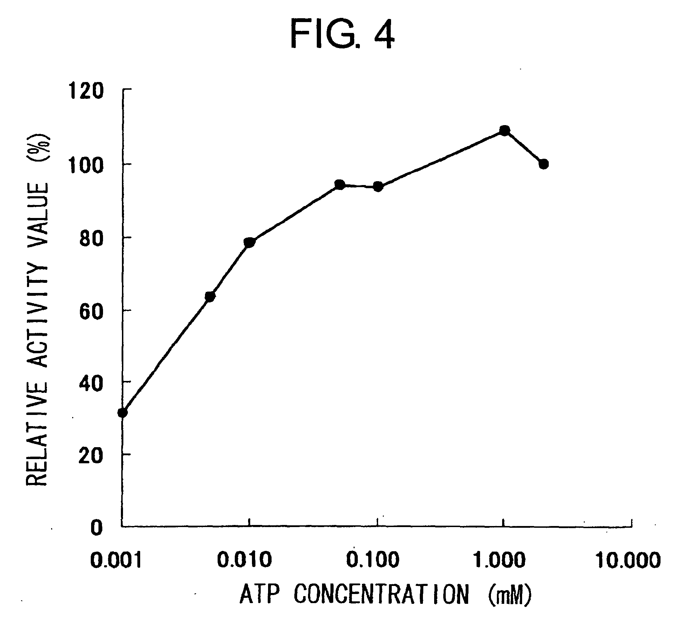

Fig. 4 shows the results of investigating changes in reactivity when the final concentration of ATP added to the phosphorylation reaction solution was changed from 1 µM to 2 mM, in the method for measuring Cdc7-ASK complex activity using GST-Mcm2 (1-130 a.a.) and Mcm2-phospho-S17 polyclonal antibody, as described above. Enzyme activity is shown as a relative activity value (%), where activity at 2 mM is taken as 100. Activity of 90% is indicated at 0.1 mM, and the reaction virtually plateaus at 1 mM. Although activity equal to or more than 30% of the maximum activity value was indicated, even in the presence of low concentrations of ATP, reactivity was clearly shown to be ATP-dependent.

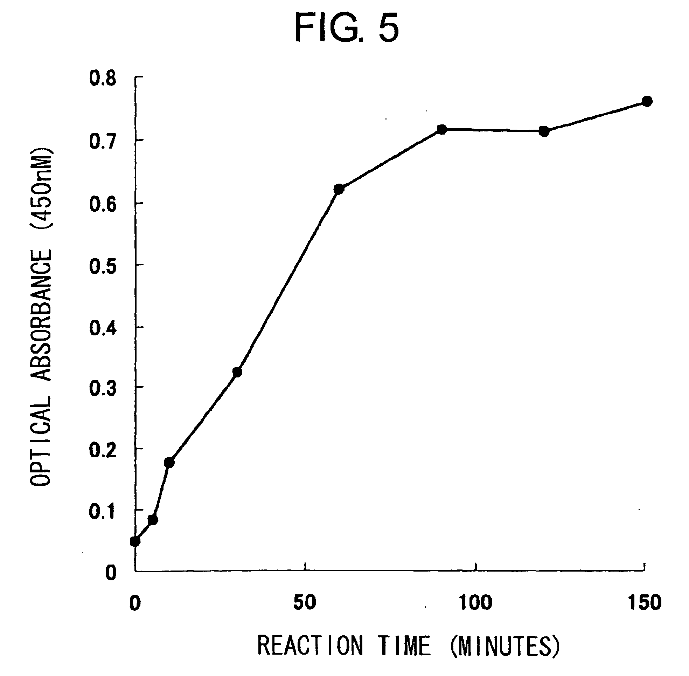

Fig. 5 shows the results of investigating changes in reactivity when the phosphorylation reaction time in the method for measuring Cdc7-ASK complex activity was changed from 0 to 150 minutes. Increases in absorbance were observed to be dependent on reaction time, and the reaction virtually plateaued at 90 minutes.

The use of other kinases in the present measurement system was examined. A GST-Mcm2 (1-130 a.a.)-sensitized plate and anti-Mcm2-phospho-S17 polyclonal antibody were used. The aforementioned Cdc7-ASK complex (wild type, WT) and inactive (KD) Cdc7-ASK complex were used as enzymes. Activity was measured for each dilution series.

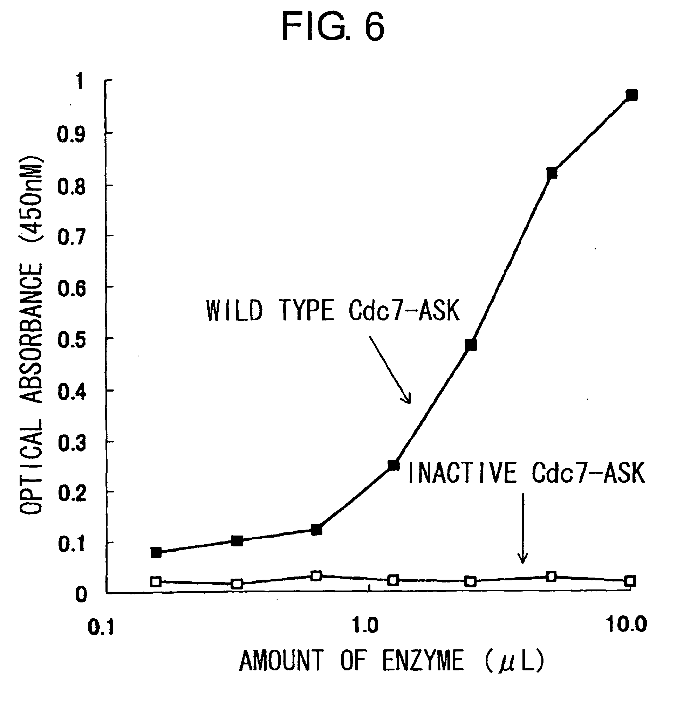

The results are shown in Fig. 6. Optical absorbance, i.e., phosphorylation of the 17th serine residue, was observed to decrease as WT Cdc7-ASK complex was diluted by up to four-fold. This was not observed for KD Cdc7-ASK complex, in which phosphorylation activity is not present. On the basis of these findings, the present measurement system was proved able to measure Cdc7-ASK complex-specific phosphorylation activity.

A radiofilter assay is a method for detecting substrate phosphorylation that uses uptake of the radioisotope [γ-³²P]ATP into an acid-insoluble fraction. It is frequently used in measuring phosphorylation activity. The sensitivity of this method and the ELISA measurement system provided in the present invention were compared.

A recombinant Cdc7-ASK complex dilution series diluted from one-fold (x1) to 64-fold in two-fold increments was prepared using a phosphorylation buffer in 1 µg of recombinant protein GST-Mcm2 (1-130 a.a.), and then adjusted to a total volume of 50 µl. After incubating at 30°C for a fixed period of time, the reaction was stopped by adding 1 ml of 10% trichloroacetic acid and 0.2% Na₄P₂O₇. The acid-insoluble fraction was trapped in a GFC filter (Whatman), and washed three times using 2% trichloroacetic acid and 0.02% Na₄P₂O₇. The radioactivity of the [γ-³²P]ATP taken up by the substrate was counted with a liquid scintillation counter.

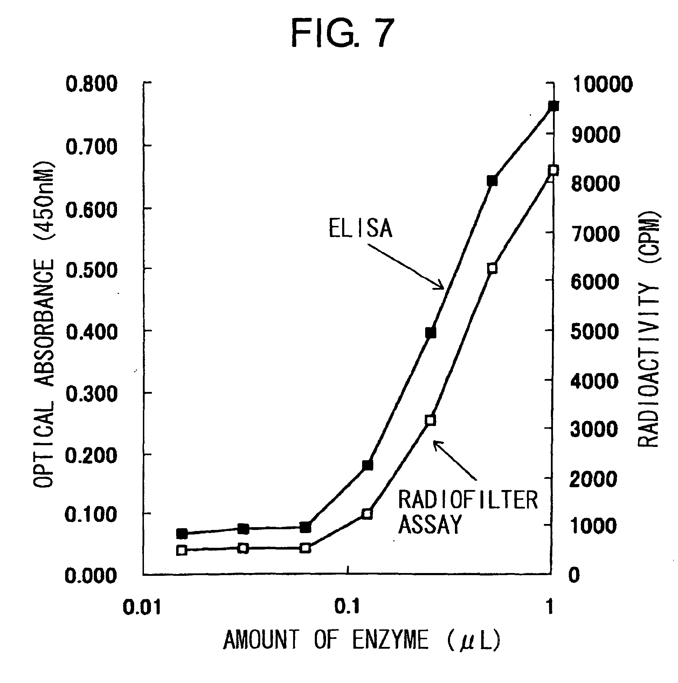

The results are shown in Fig. 7. The results are shown as relative values (%), where both the radioactive count (cpm) in the radiofilter assay, and the optical absorbance in ELISA, are taken to be 100% at the one-fold concentration of Cdc7-ASK complex enzyme. Compared to the measurement system using radioisotope [γ-³²P]ATP, the ELISA method could measure about 1/8 the amount of enzyme, since the value was 100% up to x8. In addition, while the amount of substrate used in the ELISA method is a calculated value of 0.25 µg per well, the radiofilter assay requires about 1 µg, or four times this amount. Furthermore, in the ELISA method there was hardly any background interference in the absence of enzyme. Thus, these systems are more sensitive, require smaller amounts of substrate and enzyme, and can be measured without using radioisotopes, demonstrating the superiority of the ELISA measurement systems provided by the present invention

K-252a (Calbiochem) and Staurosporine are known as kinase inhibitors. They are known as inhibitors of various kinases such as CaM kinase II, protein kinase A, protein kinase C, and protein kinase G. The ELISA systems provided by the present invention were studied using K-252a and Staurosporine. Dilution series of each inhibitor were prepared, mixed with Cdc7-ASK complex prepared with a phosphorylation assay buffer, and then adjusted to a total volume of 50 µl. This was then added to wells in which GST-Mcm2 (1-130 a.a.) was immobilized. Using an above-described method, ELISA was then used to examine Cdc7-ASK complex phosphorylation activity.

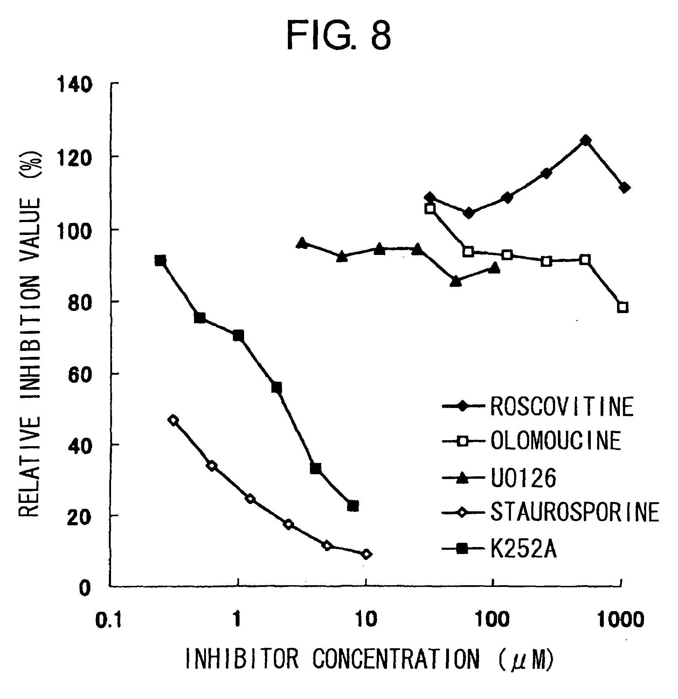

The results are shown in Fig. 8. Fig. 8 shows the relative inhibition values (%), where the activity value without an inhibitor is taken as 100. Since both K-252a and Staurosporine inhibitors are kinase inhibitors with low specificity, inhibition of phosphorylation activity by Cdc7-ASK complex was concentration-dependent. The IC50 value was 2 µM for both of these inhibitors. On the other hand, concentration-dependent inhibitory effect for Cdc7-ASK complex was not observed for Roscovitine, Olomoucine, and UO126. Roscovitine and Olomoucine are cdk2 inhibitors, and UO126 is a MAP kinase inhibitor. These kinase inhibitors were shown to lack Cdc7-ASK complex inhibition activity. Therefore, the ELISA system provided by the present invention was in fact shown to be effective in screening for agents that inhibit Cdc7-ASK complex phosphorylation activity.

### Industrial Applicability

According to the present invention, the mechanism by which the substrate protein, MCM2, is phosphorylated by Cdc7-ASK complex was elucidated. On the basis of this, substrate protein phosphorylation by the Cdc7-ASK complex could be more specifically evaluated. In other words, the methods for measuring phosphorylation effect of the present invention are useful as methods in which the phosphorylating action of Cdc7-ASK complex can be specifically evaluated. For example, the kinase activity of Cdc7-ASK has been clearly shown to increase in actively growing cancer cells (Gene, 1998, Apr 28;211(1):133-40, "A human homolog of the yeast CDC7 gene is overexpressed in some tumors and transformed cell lines.", Hess G.F., Drong R.F., Weiland K.L., Slightom J.L., Sclafani R.A., and Hollingsworth R.E., Cancer Research, Pharmacia, Upjohn, Inc., 301 Henrietta Street, Kalamazoo, MI 49001, USA). Thus, the growth ability of certain cancer cells can be predicted based on the kinase activity of Cdc7-ASK. In addition, by comparing the amount of expressed Cdc7 in numerous human cancer cells and normal cells, the present inventors also confirmed that Cdc7 is extremely strongly expressed in nearly all cultured cancer cells (including fibroblast cell lines and lymphoid lines). Thus, Cdc7-ASK kinase activity, which can be evaluated by the methods of the present invention, is useful as a carcinogenesis marker. Cdc7-ASK kinase activity can be specifically evaluated by the present invention. Thus, the growth ability of cancer cells can be evaluated more specifically.

Moreover, by applying the aforementioned measurement methods, the present invention has realized methods for evaluating the effects of test compounds on the phosphorylation function of the Cdc7-ASK complex, as well as screening methods based on these evaluation methods.

The present invention's methods for measuring Cdc7-ASK kinase activity, or of screening for compounds that regulate that activity, are useful for the development of cancer medicaments and treatment methods targeting Cdc7-ASK. Compared to using known intracellular kinases (such as Cdk-Cyclin) or proteins as targets, the use of Cdc7-ASK as a target offers the following advantages:

Firstly, by targeting Cdc7-ASK, more reliable control of activity can be expected. While Cdk-Cyclin is a family composed by numerous similar genes, Cdc7-ASK is composed of molecules whose structure is more limited. Thus, by using Cdc7-ASK as a target, activity can be reliably controlled.

Secondly, using Cdc7-ASK as a target makes it possible to specifically control cell growth. Cdc7-ASK is a factor required for the initiation and progress of the S phase. Upon loss of this activity the S phase immediately stops and cell growth is arrested. Moreover, the results of experiments using genetically manipulated mice suggest that interruption of the S phase, namely DNA replication, can potentially cause abnormal structures to accumulate in DNA, which when detected as DNA damage, can induce p53 and eventually cell death. In other words, inhibition of Cdc7-ASK activity can effectively block progression of the S phase in cancer cells, efficiently removing cancer cells by inducing cell death.

Thirdly, the use of Cdc7-ASK as a target is expected to lead to the discovery of completely new cell growth inhibitors. The structure of Cdc7-ASK kinase is unique, even among members of the kinase family. In addition, the structure of ASK is also different from that of cyclin molecules, on which considerable research has been conducted. This activation is predicted to be due to a novel, and heretofore unknown, mechanism regarding kinase activation. Thus, searching for activity inhibitors that target Cdc7-ASK is highly likely to lead to the discovery of completely new substances, undiscovered by the various types of kinase screening to date. This means that, for example, new lead compounds may be discovered in the research and development of anti-cancer agents.

Fourthly, induction of cell death can be expected when using Cdc7-ASK as a target. In response to the unexpected arrest of DNA replication, cells activate so-called check-point kinases, such as ATM, Chk1, and Cds1, which respond by delaying the progress of the cell cycle. If Cdc7-ASK inhibitors are used in combination with inhibitors against these check-point kinases, enhanced induction of cell death, due to inhibited S phase progression caused by inhibiting Cdc7-ASK activity, can be expected.

In this manner, Cdc7-ASK complex plays important roles in the initial stages of cell growth in the cell cycle. Moreover, its phosphorylation activity is proportional to the ability of cancer cells to grow. In other words, cancer cell growth can be more specifically controlled by controlling Cdc7-ASK complex activity. Thus, the screening methods of the present invention are useful as methods for obtaining compounds that can specifically control cancer cell growth.

Moreover, the screening methods of the present invention are also able to more specifically evaluate the effects of test compounds on the phosphorylation function of Cdc7-ASK complex. As a result, compounds that act more specifically on cancer can be selected.

In addition, the present invention has also provided a methods for preparing the Cdc7-ASK complex active substances required by each of the aforementioned methods. According to the methods of the present invention, complexes comprising activity similar to that of the Cdc7-ASK complex can be easily prepared in large volumes by using prokaryotic cells.

Furthermore, the present invention provides substrate proteins that are phosphorylated by the Cdc7-ASK complex. The substrate proteins of the present invention are proteins that comprise the structure required for phosphorylation by the Cdc7-ASK complex (or Cdc7-ASK complex active substance). By using such substrate proteins the phosphorylation effect of Cdc7-ASK complex can be more specifically evaluated. The Cdc7-ASK complex active substances and substrate proteins prepared based on the present invention are useful in the aforementioned methods.

In addition, the present invention has provided antibodies that identify the level of phosphorylation of substrate proteins phosphorylated by the Cdc7-ASK complex or Cdc7-ASK complex active substance. The antibodies of the present invention identify the level of phosphorylation at specific substrate protein sites. These antibodies make it possible to more specifically evaluate the phosphorylation effect of the Cdc7-ASK complex or Cdc7-ASK complex active substance. In addition, the use of the antibody of the present invention enables the phosphorylation effect of the Cdc7-ASK complex or Cdc7-ASK complex active substance to be easily evaluated based on immunoassay principles.

## Claims

1. A method for measuring the kinase activity of a Cdc7-ASK complex, comprising the following steps:
(a) contacting a substrate protein with the Cdc7-ASK complex under conditions that allow phosphorylation of the substrate protein, wherein the substrate protein is a protein comprising the amino acid sequence of SEQ ID NO: 1, or a protein functionally equivalent to that protein;
(b) measuring the level of phosphorylation of a serine residue of the substrate protein at the position corresponding to position 17 in the amino acid sequence of SEQ ID NO: 1; and
(c) measuring the kinase activity of the Cdc7-ASK complex using the level of phosphorylation as an indicator.

2. The method according to claim 1, wherein the level of phosphorylation is measured based on the level of binding of an antibody that identifies the level of phosphorylation of the serine residue.

3. The method according to claim 1, wherein the Cdc7-ASK complex is derived from a biological sample.

4. A method for measuring the effects of a test compound on the kinase activity of a Cdc7-ASK complex, comprising the following steps:
(a) contacting a test compound, a substrate protein and a Cdc7-ASK complex active substance, wherein the substrate protein is a protein comprising the amino acid sequence of SEQ ID NO: 1, or a protein functionally equivalent to that protein, and wherein they are contacted in any of the following orders i) to iii):
i) the test compound and substrate protein are contacted, followed by contacting the Cdc7-ASK complex active substance,
ii) the substrate protein and Cdc7-ASK complex active substance are contacted in the presence of the test compound, or
iii) the substrate protein and Cdc7-ASK complex active substance are contacted, followed by contacting the test compound;
(b) measuring the level of phosphorylation for a serine residue of the substrate protein at the position corresponding to position 17 in the amino acid sequence shown in SEQ ID NO: 1; and
(c) measuring the effect of the test compound on the kinase activity of the Cdc7-ASK complex active substance using the level of phosphorylation as an indicator.

5. A method of screening for compounds comprising the effect of regulating the kinase activity of a Cdc7-ASK complex, comprising the following steps:
(a) measuring the effect of a test compound on the kinase activity of the Cdc7-ASK complex according to the method described in claim 4; and
(b) selecting a test compound with a high or low level of phosphorylation by comparison with a control that has not been contacted with the test compound.

6. A screening method according to claim 5, wherein a compound having a low level of phosphorylation is selected in step (b) of claim 5.

7. An inhibitor of cell growth comprising a compound selected according to the screening method of claim 6 as its active ingredient.

8. A kit for measuring the kinase activity of a Cdc7-ASK complex comprising:
(a) a substrate protein comprising a continuous amino acid sequence that comprises a serine residue at position 17 of the amino acid sequence of SEQ ID NO: 1, and that which is selected from the amino acid sequence of SEQ ID NO: 1; and
(b) an antibody that identifies the level of phosphorylation of the serine residue of the substrate protein at the position corresponding to position 17 of the amino acid sequence of SEQ ID NO: 1.

9. A kit for evaluating the effect of a test compound on the kinase activity of a Cdc7-ASK complex, comprising:
(a) a Cdc7-ASK complex active substance; and,
(b) a substrate protein comprising a continuous amino acid sequence that comprises the serine residue at position 17 of the amino acid sequence of SEQ ID NO: 1, and is selected from this amino acid sequence.

10. A process for producing a Cdc7-ASK complex active substance comprising the following steps:
(a) introducing a DNA encoding human Cdc7 protein and a DNA encoding a protein comprising the amino acid sequence described in SEQ ID NO: 10 or a protein functionally equivalent to the protein, into prokaryotic cells in a state that allows monocistronic expression;
(b) expressing the two DNAs; and
(c) recovering the expressed protein.

11. An antibody that identifies the level of phosphorylation of the serine residue at position 17 of a protein comprising the amino acid sequence of SEQ ID NO: 1.

12. A protein according to any of the following (a) to (d):
(a) a protein comprising the amino acid sequence of SEQ ID NO: 1;
(b) a protein comprising a continuous amino acid sequence that is selected from the amino acid sequence described in SEQ ID NO: 3, and comprises the serine of position 17;
(c) a protein comprising an amino acid sequence in which one or more amino acids in the amino acid sequence of SEQ ID NO: 1 are substituted, deleted, added and/or inserted, wherein the protein is phosphorylated by human Cdc7-ASK complex; and
(d) a protein comprising an amino acid sequence comprising 90% or more homology with the amino acid sequence of SEQ ID NO: 3, wherein the protein is phosphorylated by human Cdc7-ASK complex.

13. A protein comprising a continuous amino acid that comprises the amino acid sequence of SEQ ID NO: 10 and that which is selected from the amino acid sequence of SEQ ID NO: 9.

14. A polypeptide according to claim 13 that comprises the amino acid sequence of SEQ ID NO: 10.
